# NOUVEAU FASCICULE DE BREVET EUROPEEN

(11) **EP 0 517 833 B2**
(45) Date de publication et mention de la décision concernant l'opposition: **16.07.2008**
(45) Mention de la délivrance du brevet: 02.11.1995
(21) Numéro de dépôt: 91906203.4
(22) Date de dépôt: 01.03.1991
(51) Int. Cl.: C12N 15/82, C12N 5/10, A01H 5/00, C12N 15/40

(54) **TRANSFORMATION GENETIQUE ET REGENERATION DE LA BETTERAVE SUCRIERE**
REGENERIERUNG UND GENETISCHE TRANSFORMATION DER ZUCKERRÜBE
REGENERATION AND GENETIC TRANSFORMATION OF SUGAR BEET

(30) Priorité: 02.03.1990 FR 9002686; 02.03.1990 FR 9002687
(43) Date de publication de la demande: 16.12.1992
(73) Titulaire: Bayer BioScience N.V., 9052 Gent (BE)
(72) Inventeur: GERENTES, Denise, F-63170 Aubière (FR); PEREZ, Pascual, F-63110 BEAUMONT (FR); KALLERHOFF, Jean, F-63400 Chamalières (FR); BEN TAHAR, Sophie, F-63000 Clermont-Ferrand (FR); PERRET, Jo¬l, F-63540 Romagnat (FR)
(74) Mandataire: Almond-Martin, Carol
(86) Numéro de dépôt international: PCT/FR1991/000170
(87) Numéro de publication internationale: WO 1991/013159

(56) Documents cités:
- EP-A- 0 223 452
- EP-A- 0 240 208
- EP-A- 0 257 993
- J. BOTTERMAN ET AL: "Discovery, transfer to crops, expression and biological significance of a bialaphos resistance gene." BRITISH CROP PROTECTION COUNCIL, (PROPECTS AMINO ACID BIOSYNTH. INHIB. CROP PROT. PHARM. CHEM.), vol. 42, 1989, pages 63-68, XP001189239
- F. MEULEWAETER ET AL : "Structural analysis of the coat protein gene in different BNYVV isolates." MED.. FAC. LANDBOUWW. RIJKSUNIV. GENT, vol. 54, no. 2b, 1989, pages 465-468, XP001189253
- M.H. HARPSTER ET AL : "Relative strenghts of the 35S califlower mosaic virus, 1;2; and nopaline synthase promoters in transformed tobacco sugarbeet and oilseed rape callus tissue." MOL. GEN. GENET., vol. 212, no. 1, 1988, pages 182-190, XP001189234
- C.A. WOZNIAK ET AL: "Transformation of sugarbeet cell suspension cultures mediated by Agrobacterium tumefacines." JOURNAL OF CELLULAR BIOCHEMISTRY, vol. suppl 13D, 27 mars 1989 (1989-03-27) - 7 avril 1989 (1989-04-07), page 272 XP001189232 R. LISS, INC. NEW YORK, US
- J.W. SAUNDERS ET AL : "One step shoot regeneration from callus of whole plant leaf explants of sugarbeet lines and a somaclonal variant for IN VITRO behavior." JOURNAL PLANT PHYSIOL., vol. 124, no. 5, 1986, pages 473-479, XP001149464
- J. KALLERHOFF ET AL : "Beet necrotic yellow vein virus coat protein-mediated protection in sugarbeet (Beta vulgaris L.) protoplasts." BIOLOGICAL ABSTRACTS, vol. 90, no. 10, 1990, XP001189233 & PLANT CELL REP., vol. 9, no. 4, 1990, pages 224-228,
- J.C. THOMAS ET AL : "Insect protease inhibitor expression in transgenic tobacco and cotton." ABSTRACT VIITH INTERNATIONAL CONGRESS PLANT TISSUE AND CELL CULTURE, 24 - 29 juin 1990, page 78, XP008030733 AMSTERDAM , NL.
- LOWELL OWENS: "Optimization of sugarbeet leaf-disc culture for induction of regenerative wound callus." ANNUAL MEETING OF THE AMERICAN SOCIETY OF PLANT PHYSIOLOGISTS, 10 - 14 juillet 1988, page 108, XP008030664 RENO, NEVADA; US: & PLANT PHYSOL, vol. 86, no. 4 suppl., 1988, page 108. (Bethesda, US. )
- BOUZOUBAA S ET AL: "NUCLEOTIDE SEQUENCE OF BEET NECROTIC YELLOW VEIN VIRUS RNA-2" JOURNAL OF GENERAL VIROLOGY, READING, BERKS, GB, vol. 67, 1986, pages 1689-1700, XP001149481 ISSN: 0022-1317
- DIXON B: "DENMARK OKS TRIAL OF ENGINEERED BEETS" BIO/TECHNOLOGY, NATURE PUBLISHING CO. NEW YORK, US, vol. 7, octobre 1989 (1989-10), page 1001 XP001107144 ISSN: 0733-222X

## Description

L'invention concerne un procédé de transformation génétique de cellules végétales appartenant à l'espèce Beta vulgaris, suivie éventuellement par une étape de régénération de cellules transformées en plante entière.

L'invention concerne également les cellules transformées, les bourgeons, les plantes et les graines transgéniques susceptibles d'être produits par ce procédé.

L'invention concerne en outre, des plantes transgéniques appartenant à l'espèce Beta vulgaris, résistantes à l'infection par le virus des nervures jaunes et nécrotiques de la betterave à sucre (BNYVV), exprimant la protéine de capside du BNYVV, ou un dérivé de cette protéine.

Les références bibliographiques apparaissant dans la description de l'invention sont répertoriées sous forme de bibliographie.

L'obtention de plantes transgéniques met en oeuvre le transfert du fragment d'ADN sélectionné dans la cellule végétale, la sélection des cellules transformées de façon stable et la régénération de plantes entières à partir des cellules sélectionnées transformées.

Le problème technique gui s'est posé lors de l'élaboration de la présente invention était de trouver une méthode de transformation de cellules de betterave présentant une fréquence de transformation élevée qui pouvait être associée avec succès à une méthode de régénération de plante transgénique. A ce jour, une telle méthode de transformation et de régénération n'a pas été décrite, empêchant la production de betteraves transgéniques présentant des caractéristiques agronomiquement intéressantes, telle que la résistance à la rhizomanie.

Actuellement, il existe deux grandes voies de transfert d'ADN dans les cellules végétales.

La première voie est une voie physico-chimique (électroporation, microinjection, polycations, canon à particules). Chez la betterave, des cellules transformées de manière stable ont été sélectionnées après électroporation d'un gène codant pour la résistance à la kanamycine dans les protoplastes (Lindsey et al, 1989).

Cependant, ce procédé n'a jamais pu conduire à la création de betteraves transgéniques puisque la régénération de plantes à partir de protoplastes n'a pu être obtenue chez cette espèce.

La deuxième voie de transfert d'ADN est une voie biologique utilisant comme vecteur une bactérie du sol : Agrobacterium tumefaciens ou rhizogenes.

Différentes souches de ces deux espèces ont été utilisées avec succès pour la transformation de cellules de betteraves. Ces cellules transformées ont donné naissance soit à des tumeurs avec Agrobacterium tumefaciens (Krens et al, 1988) soit à des racines avec Agrobacterium rhizogenes (Yacoub et al, 1987). Ces tissus transformés (racines et tumeurs) n'ont jamais permis à ce jour la régénération de plantes chez la betterave. Ceci vient du fait que ces deux phénotypes sont les résultats de l'intégration dans le génome de la cellule non seulement du fragment d'ADN désiré mais aussi d'un fragment d'ADN de la bactérie qui perturbe l'équilibre hormonal de la cellule (Akiyoshi et al, 1983). Pour pallier ces problèmes, ces gènes ont été délétés, donnant de nouvelles souches d'Agrobacterium dites souches désarmées.

L'utilisation des souches d'Agrobacterium désarmées implique l'association d'un système sélectif pour la transformation de cellules végétales. Le succès dans l'obtention de transformants est étroitement lié à la mise au point d'un bon système sélectif. Le gène sélectif le plus utilisé dans ce domaine est celui provenant du transposon Tn5 d'Escherichia coli (Rothstein et al, 1981) codant pour la néomycine phosphotransférase (NPT II) qui confère la résistance à la kanamycine (An et al, 1985).

La plupart des vecteurs plasmidiques utilisés dans ces souches désarmées contiennent dans l'ADN transférable, outre le gène désiré, le gène NPT II, sous le contrôle de signaux de transcription végétaux (Bevan, 1984 ; An, 1986). Le système de sélection comprend d'une part le gène conférant la résistance, et d'autre part, l'agent sélectif. Ceci implique de déterminer les concentrations d'agent sélectif permettant à la fois de tuer les cellules non transformées et de laisser croître les cellules transformées. Les seuls travaux publiés concernant la sélection de cellules de betterave après transformation d'explants pluricellulaires par Agrobacterium font appel à un autre système sélectif : hygromycine B / hygromycine B phosphotransferase (Harpster et al, 1988).

La transformation par Agrobacterium nécessite dans un premier temps le choix d'un type de cellule ou d'un type d'explant qui va faire l'objet de la transformation. Par exemple, des explants tels que des hypocotyles, des morceaux de feuilles (Krens et al, 1988) peuvent être transformés par Agrobacterium.

La fréquence de transformation peut varier selon le type de cellule ayant fait l'objet de la transformation, cette variabilité étant souvent d'une nature imprévisible.

Lors de la production d'une plante transgénique, la transformation est suivie par un procédé de régénération.

L'efficacité d'obtention des plantes transgéniques dépend de la fréquence de régénération de plantes à partir de cellules transformées d'une part, et la fréquence de transformation des cellules d'autre part.

Par exemple, la transformation de tronçons de pétioles de betterave a donné des cals transformés sélectionnés sur kanamycine. Cependant, il n'a jamais été possible de régénérer des plantes à partir de ces cals transformés issus de pétioles, bien que la régénération d'embryons somatiques et de bourgeons à partir de ce type de cal dans l'état non-transformé ait été reportée (Tetu et al, 1987). Cet échec n'est pas surprenant si on prend en compte la faible fréquence de régénération décrite.

Ceci traduit bien les problèmes de régénération, à partir d'explants, de plantes non-transformées, rencontrés depuis longtemps chez la betterave sucrière (Ritchie et al, 1989). Plusieurs auteurs ont décrit la régénération directe de bourgeons adventifs à partir de fragments de pétioles de plantes en multiplication végétative (Detrez et al, 1988 ; Freytag et al, 1988). Bien que ce phénomène se soit avéré reproductible dans des conditions appliquées par les inventeurs, la fréquence s'est révélée beaucoup trop faible pour être associée à la transformation. D'autres part, il semble que ces néoformations proviennent de massifs cellulaires non accessibles à la bactérie et apparemment peu sensibles à un agent sélectif.

Une autre technique de régénération de plantes non transformées à partir de cellules de betteraves à sucre est celle décrite par Saunders et al (1986). Elle met en jeu dans un premier temps, l'induction de cals friables indépendants d'hormones et initiés probablement à partir des cellules épidermiques du limbe, puis dans un deuxième temps, la régénération de bourgeons et d'embryons somatiques à partir de ces cals. Cette technique a été facilement reproductible sur plusieurs variétés de betteraves sucrières. Un des intérêts de ce processus est la possibilité d'obtenir aisément des suspensions cellulaires à partir de cals friables dont le potentiel organogène peut être entretenu pendant quelques mois.

Les inventeurs ont découvert que ce matériel organogène, c'est-à-dire les cals friables, peut être utilisé, dans des conditions précises, pour la transformation par Agrobacterium tumefaciens.

Il y a encore peu de temps la transformation de suspensions cellulaires ne paraissait pas concevable selon le dogme établi que le transfert d'ADN par Agrobacterium dans une cellule végétale nécessitait des lésions cellulaires. Cependant, des auteurs ont reporté la transformation de cellules en suspension chez le tabac et la carotte (AN, 1985 ; Scott et Draper, 1987). A ce jour, la transformation de cellules en suspension chez la betterave n'a pas été décrite. Par ailleurs, il est à noter que les méthodes de transformation qui s'appliquent avec succès à une espèce végétale ne peuvent pas être étendues systématiquement à d'autres espèces. Les conditions de transformation, les matériels de départ et les milieux de culture sont des paramètres variables spécifiques à chaque espèce.

En ce qui concerne la transformation et la régénération de betteraves transgéniques résistantes à la rhizomanie, tout progrès a été empêché, par le manque d'un procédé de production fiable.

Le virus des nervures jaunes et nécrotiques de la betterave à sucre (BNYVV) est un virus à composants multiples, constitué de particules virales à symétrie hélicoïdale, contenant quatre types de RNA simple brin, de polarité positive (Putz, 1987). Ce virus est disséminé par un champignon du sol, Polymixa betae, qui parasite les cellules superficielles des radicelles de chénopodiacées, dont fait partie la betterave à sucre. Cette chénopodiacée bisannuelle reste à l'état de rosette la première année en donnant une racine charnue et sucrée, et monte à graine la deuxième année après vernalisation. La persistance de la maladie dans le sol est due aux kystes formés par le champignon (Tamada, Baba, 1973).

Il est connu que le virus se développe essentiellement dans la partie racinaire (pivot et racines secondaires). Le symptôme principal de la maladie consiste en une prolifération du chevelu racinaire (d'où le nom de rhizomanie). Mais le nom du virus provient en fait de symptômes plus tardifs visibles dans la partie végétative à savoir : nécroses et jaunissement des nervures dus au fait que le virus se développe surtout au niveau des vaisseaux racinaires, en perturbant ainsi le métabolisme de toute la plante (Sallé et al, 1986).

Plusieurs auteurs ont reporté que le virus n'est détecté dans la partie aérienne que très rarement alors qu'il est présent en grande quantité au niveau des racines et du pivot (Putz, 1977 ; Ziegler et al, 1985).

Le contrôle des maladies virales des végétaux reste problématique, malgré l'avènement du génie génétique végétal.

Abel et al (1986) ont introduit un gène codant pour la protéine de capside du virus de la mosaïque du tabac (VMT) dans le génome des plantes naturellement sensibles à ce virus. Cette manipulation génétique a provoqué un retard du développement de la maladie chez les plantes transgéniques. D'autres réalisations du même type ont été reportées avec d'autres virus ;
- Alfalfa Mosaic Virus, et Tobacco Rattle Virus, sur tabac (Van Dun et al, 1987),
- Alfalfa Mosaic Virus sur tabac (Loesch Fries et al, 1987),
- Alfalfa Mosaic Virus sur tabac et tomate (Tumer et al, 1987).

Deux équipes ont créé des plantes transgéniques exprimant des gènes codant pour des ARN antisens, complémentaires de l'ARN codant pour la protéine de capside, et ils montrent que la résistance est beaucoup moins importante que celle conférée par la protéine de capside (Cuozzo et al, 1988 ; Hemenway et al, 1988).

Enfin, d'autres laboratoires ont créé des plantes transgéniques exprimant des gènes codant pour des ARN satellites. Cette stratégie a été adoptée par Gerlach et al (1987) pour le virus des tâches annulaires du tabac, et par Harrison et al (1987) pour le virus de la mosaïque du concombre (CMV).

Aucun satellite n'étant connu pour le BNYVV, les inventeurs ont envisagé de faire produire aux cellules végétales transformées soit des ARN antisens, soit des ARN sens codant pour des protéines virales normales, mutées ou délétées, susceptibles d'inhiber le développement du virus.

Le procédé de transformation de l'invention implique :
- la transformation de cals friables en dispersion donnant des cals, des suspensions cellulaires et des plantes transformés.

Plus particulièrement, la présente invention concerne un procédé de transformation de cellules végétales appartenant à l'espèce Beta vulgaris **caractérisé en ce qu**'il comprend la mise en contact d'une dispersion de cals blancs friables dans un milieu de culture cellulaire végétale liquide contenant 0 à environ 3.0 mgL⁻¹ d'une cytokine, avec Agrobacterium tumefaciens contenant un vecteur portant un gène destiné à être introduit dans les cellules végétales, suivie de coculture des cellules végétales et des bactéries pour donner lieu à des cals friables transformés. Selon un mode de réalisation préféré de l'invention, ce procédé de transformation comprend les étapes successives suivantes
I) induction de cals blancs friables à partir d'explant ;
II) dispersion des cals dans un milieu de culture cellulaire végétale liquide contenant 0 à environ 3.0 mgl⁻¹ d'une cytokinine ;
III) mise en contact de la dispersion avec Agrobacterium tumefaciens contenant un vecteur portant un gène destiné à être introduit dans les cellules végétales, suivie de coculture des cellules végétales et des bactéries ;
IV) lavage des cellules végétales pour éliminer les bactéries et sélection des cellules transformées sur un milieu sélectif ;
V) culture des cellules transformées sélectionnées pour obtenir des cals friables transformés.

La première étape de la transformation est l'induction de cals blancs friables à partir d'explants de betterave. Les explants qui peuvent servir dans cette étape peuvent être par exemple des disques de feuilles, des tronçons de pétioles, etc. De préférence, les explants sont des morceaux de jeunes feuilles prélevées d'une plante âgée de moins de trois mois.

Par exemple, après germination de graines de betterave d'environ un mois, de jeunes feuilles de 3 à 5 cm de long sont prélevées de chaque plante et sont soumises à une étape de désinfection et rinçage. Chaque feuille est ensuite découpée en petits morceaux de 0,25 cm² à 1.0 cm². Les feuilles peuvent être prélevées de la plante jusqu'à deux mois environ après les premiers prélèvements. Après cette période, l'aptitude de régénération des feuilles diminue. Les disques de feuilles sont ensuite mis en culture sur un milieu de culture cellulaire végétale contenant de 0,1 à 5,0 mgl⁻¹ cytokinine. De préférence, la cytokinine est présente à raison d'environ 1.0 mgl⁻¹ et peut être, par exemple, la 6-buzylaminopurine (BAP), la zéatine, ou la kinétine. La BAP est particulièrement préférée. Le milieu de culture cellulaire végétale est avantageusement le milieu de Murashige et Skoog (1962), dit milieu M.S. Les explants sont cultivés à 30°C environ pendant 30 jours dans l'obscurité, et ensuite ils sont sortis en chambre de culture avec une photopériode de 18/24 H par exemple à environ 25°C le jour et 20 ° C la nuit.

De 4 à 10 semaines après la mise en culture, des cals blancs friables apparaissent autour, sur ou sous les explants foliaires.

L'étape suivante du procédé de transformation est la production d'une dispersion des cals dans un milieu de culture liquide. Cette dispersion sera utilisée ultérieurement pour la transformation.

La production d'une dispersion des cals s'effectue par dispersion des cals apparus depuis 2 à 6 semaines sur tes explants, dans un milieu de culture liquide contenant 0 à 3,0 mgl⁻¹ cytokinine, par exemple le milieu MSB1. Deux types cellulaires, habitué et noduleux, peuvent être observés dans les dispersions, notamment :
- type habitué (A) : suspension fine, verte à croissance rapide, et qui régénère ponctuellement des formations vitrifiées se développant difficilement ;
- type noduleux (C) : suspension d'agrégats compacts jaunâtres, à croissance plus lente, régénère plus fréquemment que la première, des structures embryonnaires compactes se développant assez bien.

Ce type est aussi connu sous le nom "type régénérant". Les cals en dispersion peuvent être examinés avant la transformation dans le but de séparer les deux types cellulaires, c'est-à-dire noduleux et habitué. Un examen visuel des cals permet de repérer les deux types qui sont ensuite enlevés du milieu avec une pince et redispersés. Les suspensions cellulaires soigneusement initiées à partir de dispersions de cals blancs friables noduleux se sont avérées être le matériel idéal pour optimiser l'efficacité de la transformation.

Chacun des deux types cellulaires peuvent être soumis à la transformation mais il est préférable de transformer le type noduleux si ta régénération de la plante est désirée ultérieurement.

La dispersion des cals est ensuite mise en contact avec la bactérie Agrobacterium tumefaciens.

Le protocole de transformation est effectué sur les dispersions. Un échantillon des bactéries dans du milieu frais est ajouté à la dispersion des cellules de betterave. La coculture des cellules végétales et des bactéries se fait à l'obscurité pendant trois jours environ, en chambre de culture.

L'Agrobacterium tumefaciens utilisé dans la transformation contient un vecteur portant un gène destiné à être introduit dans les cellules de betterave. Les souches utilisées par les inventeurs contiennent des vecteurs binaires portant le gène d'intérêt. Les trois souches d'Agrobacterium tumefaciens désarmées utilisées dans le travail décrit ici sont LBA 4404 (Hoekema et al, 1983 ; EHA 101 (Hood et al, 1986); C58'3 (Dale et al, 1989). Bien évidemment, le gène d'intérêt est placé sous le contrôle de signaux régulateurs appropriés, par exemple un promoteur permettant son expression dans la cellule végétale et, le cas échéant, dans la plante transgénique régénérée. Le promoteur peut être choisi pour permettre l'expression spécifique du gène dans une certaine partie de la plante, ou à un certain stade de son développement. En revanche, un promoteur constitutif peut être utilisé, donnant lieu à l'expression ubiquitaire du gène introduit.

Comme gène, on peut citer des gènes codants pour des caractères agronomiques intéressants, par exemple la résistance aux herbicides, aux insectes et aux virus, ou encore un gène induisant la stérilité mâle. La résistance aux herbicides peut être conférée, par exemple par le type de gène décrit par De Block et al (1987) et par Bedbrook et al (1988). Un gène susceptible de conférer une résistance aux insectes est le gène de la protéine crystalline de B. thiuringiensis (Perlak et al (1990) ; Vaeck et al (1987) ; Fischoff D, et al (1987)) La stérilité mâle peut être induite par un gène codant pour un ribonucléase, tel que celui décrit par Mariani et al (1990). La résistance aux virus peut parfois être induite par le gène codant pour la protéine de capside du virus en question. Par exemple, la résistance au virus BWYV peut être conférée par le gène décrit par Gielen et al (1990). Par ailleurs, une protection contre le BNYVV, responsable de la rhizomanie, peut également être induite par ce même type de gène. Selon le procédé de l'invention, ce gène codant pour cette protéine de capside peut être introduit dans des cellules de betteraves, conférant ainsi la résistance à la rhizomanie. Les vecteurs décrits dans ces exemples peuvent être utilisés pour l'introduction de ces gènes.

Le vecteur porte également un gène codant pour une protéine permettant la sélection des transformants, par exemple la néomycine phosphotransférase (NPT II) qui confère la résistance à la kanamycine. De plus, un gène "reporteur" peut être introduit dans le vecteur afin de pouvoir confirmer le caractère transformé du tissu végétal. Un exemple d'un tel gène reporteur est le gène uid A d'E. coli codant pour l'enzyme β-glucuronidase. Le dosage de l'activité enzymatique de cette protéine se fait facilement avec des substrats chromogènes ou fluorescents.

Après la coculture des cellules de betteraves et des bactéries, une étape de lavage avec un agent bactériostatique est effectué pour éliminer les bactéries. Comme agent bactériostatique, la cefotaxime peut être utilisée. Le lavage peut être effectué en deux étapes, par exemple une première fois avec le milieu MSB1 contenant 600 mgl⁻¹ cefotaxime, puis une deuxième fois dans du milieu MSB1 contenant 300 mgl⁻¹ cefotaxime.

Ensuite, la sélection des cellules transformées est effectuée grâce au gène codant pour la résistance à l'agent sélectif. Les cellules lavées sont donc mises en culture pendant une quinzaine de jours sur un milieu contenant l'agent sélectif, par exemple la kanamycine et l'agent bactériostatique. Les cellules végétales sont ensuite repiquées sur du milieu frais.

Trois à huit semaines après la coculture, des cals blancs apparaissent. Ces cals transformés présentent les deux types cellulaires, noduleux et habitué.

Les cals transformés ainsi obtenus ont, comme caractéristique physique principale, qu'ils peuvent être dispersés en liquide par une agitation relativement douce et celà, dès qu'ils ont une taille de 3 à 5 mm. D'une manière surprenante, ces cals transformés conservent la nature blanche, friable et régénérante qu'ils possédaient avant la transformation, et s'avèrent donc être un matériel approprié pour la régénération de plantes transgéniques. Il est à noter que la transformation effectuée sur explants, et non sur cals (Harpster et al, 1988), donne lieu à des cals transformés très compacts qui ne présentent pas de caractère friable et qui ne sont pas régénérants.

Quand ils sont suffisamment développés, les cals transformés de l'invention sont repiqués sur un milieu MSB1 avec de la céfotaxime et éventuellement de la kanamycine.

D'une manière surprenante, il a été constaté qu'un mois après le clonage des cals transformés, la kanamycine et la céfotaxime peuvent être supprimés du milieu sans que la bactérie ne se développe. Ceci peut présenter un avantage pour l'étape de régénération, les cellules n'étant plus en contact avec les antibiotiques.

Après l'obtention des cals friables transformés, la régénération de la plante transgénique peut être initiée, en commençant par la régénération de bourgeons et/ou d'embryons transgéniques.

Le procédé de régénération de bourgeons et/ou d'embryons transgéniques selon l'invention est caractérisé par l'obtention de cals friables transformés selon le procédé décrit ci-dessus, suivie du repiquage des cals friables transformés sur un milieu de culture, par exemple le milieu M.S, contenant 0 à 3,0 mgl⁻¹ d'une cytokinine et éventuellement un agent bactériostatique et un agent sélectif. Comme cytokinine, on peut citer la zéatine, la kinétine et la BAP, par exemple à une concentration de 1 mgl⁻¹. La BAP est particulièrement préférée.

Des bourgeons et/ou embryons régénèrent sur certains des cals après des délais variant d'une semaine à plusieurs mois.

A partir de ces bourgeons et/ou embryons transgéniques, il est possible, selon l'invention, de régénérer des plantes par repiquage des bourgeons et/ou embryons sur un milieu de culture tel que le milieu M.S contenant un cytokinine à faible concentration, par exemple entre 0,05 et 0,15 mgl⁻¹, de préférence 0,1 mgl⁻¹. La BAP est préférée dans cette étape. Les structures régénérées commencent alors à développer des feuilles et elles sont remises en multiplication végétative en pots ou boîtes. Les bourgeons les plus développés sont alors mis sur un milieu d'enracinement tel que le milieu MS contenant de l'acide naphtalène-acétique, par exemple 1 mgl⁻¹. Les racines apparaissent 2 à 6 semaines après. Les plantes peuvent alors être acclimatées en serre.

L'invention concerne également la production de graines à partir des plantes transgéniques par vernalisation de ces plantes transgéniques et récolte des graines après floraison.

L'invention concerne également les cals friables transformés, les bourgeons et/ou embryons transformés, les plantes transgéniques et les graines transgéniques de ces plantes susceptibles d'être produits selon les procédés décrits ci-dessus.

L'invention concerne également des graines de plantes transgéniques qui comprennent des graines transgéniques.

La présente invention concerne également la transformation génétique et la régénération de la betterave à sucre (Beta vulgaris ssp saccharifera) dans le but de créer des plantes résistantes à la rhizomanie.

Plus particulièrement, les inventeurs ont transformé des cellules végétales par des séquences codant pour la protéine de capside du BNYVV, ou pour des variantes de celle-ci.

La mise en oeuvre de cet aspect de l'invention a consisté à :
- construire par les techniques de recombinaison génétique in vitro, des gènes artificiels, potentiels de résistance au virus des nervures jaunes et nécrotiques de la betterave à sucre (BNYVV : Beet Necrotic Yellow Vein Virus) provoquant la rhizomanie,
- transférer de façon stable ces gènes de résistance dans des betteraves à sucre.

Le gène codant pour la protéine de capside du BNYW a été localisé et séquencé (Bouzoubaa et al, 1986). Cependant, il ne pouvait pas être prévu que l'expression de cette protéine dans des cellules de betterave inhiberait le développement du virus. Le virus étant transmis par le champignon du sol Polymixa betae, la voie d'infectabilité n'est pas comparable à celle des virus pour lesquels la protéine de capside s'est montré protectrice. D'autre part, la protéine de capside du BNYVV est codée par l'extrémité 5'du ARN2, ce qui veut dire que la protéine peut être traduite dans la cellule infectée immédiatement après l'infection. Pour d'autres virus, la protéine de capside est codée par un ARN subgénomique et est donc produite plus tard dans le cycle de l'infection. Pour ces raisons, l'effet protecteur présenté par la protéine de capside pour d'autres virus végétaux ne pouvait pas être prévu chez le BNYVV.

De plus, les inventeurs ont constaté plusieurs phénomènes inattendus : premièrement, il a été observé que des cellules transformées par une même séquence codant pour la protéine de capside de 22 Kd (nucléotides 145 à 708) et au moins une partie de la protéine de 75 Kd qui est composée de la protéine de 22 Kd fusionnée avec la protéine 54 Kd (nucléotides 709 à 2218), donne lieu à l'expression de deux protéines dans la même cellule, c'est-à-dire la protéine de 22 Kd et une protéine chimérique composée de la protéine de 22 Kd additionnée de la partie de la protéine de 75 Kd codée par la séquence transformante. Cette expression est le résultat du phénomène de "readthrough", le codon "stop" à la fin de la protéine de 22 Kd étant parfois supprimé dans la cellule par des tRNA suppresseurs. Il a été constaté que le taux d'expression de la protéine chimérique de "readthrough" est particulièrement élevé, et pourrait jouer un rôle dans la résistance conférée à la cellule exprimant les deux protèines à la fois.

Deuxièmement, les inventeurs ont observé que dans des plantes transgéniques les protéines exprimées selon l'invention sont exprimées spécifiquement dans les racines, et ce malgré l'utilisation de promoteurs constitutifs.

Ce phénomène est totalement inattendu et présente plusieurs avantages pour la plante. Tout d'abord, les cellules cibles du BNYW sont spécifiquement protégées. De plus, l'absence d'expression de la protéine de capside et de ses dérivés dans les parties aériennes de la plante, partie qui n'est pas susceptible d'être infectée par le BNYVV, représente une économie énergique importante pour la plante. Par ailleurs, l'expression dans les racines, à l'exclusion de toute expression dans d'autres parties de la plante, est un phénomène qui n'aurait pas pu être obtenu en utilisant un promoteur dit "spécifique" pour les racines. Ce genre de promoteur conduit, en fait, à une expression plus importante dans les racines, et une expression faible dans les autres parties de la plante. Des expériences, effectuées par les inventeurs, utilisant un gène marqueur (GUS) ont démontré que l'expression spécifique n'est pas dû à une mauvais fonctionnement du promoteur, puisque le produit d'expression du gène GUS est exprimée dans toutes les parties de la plante, lorsqu'elle est sous le contrôle de ces mêmes promoteurs. L'expression spécifique pourrait être le résultat de l'instabilité de la protéine dans les parties aériennes de la plante, ou à une mauvaise traduction. Ces expériences montrent que le promoteur ne semble pas jouer de rôle dans l'expression spécifique.

Il a également été constaté que le promoteur 35S a une efficacité de transcription de 30 à 50 fois plus élevée que le promoteur Nos dans des cellules de betterave. L'invention concerne des plantes transgéniques produisant :
- la protéine de capside du BNYVV,
- des protéines de capside modifiées définies ci-dessous (acides aminés supplémentaires, acides aminés permutés, acides aminés délétés), qui conservent l'effet protecteur vis-à-vis du BNYVV
- des ARN sens et des ARN antisens de différentes tailles et dirigés contre différentes régions de l'ARN2 du BNYVV.

Plus particulièrement, cet aspect de l'invention concerne une plante transgénique appartenant à l'espèce Beta vulgaris et résistante à l'infection par le virus des nervures jaunes et nécrotiques de la betterave à sucre (BNYVV), ladite plante étant transformée d'une manière stable par un fragment d'acide nucléique, dont le produit d'expression est capable de conférer ladite résistance, ledit fragment étant dérivé de l'extrémité 5' de l'ARN2 génomique ou subgénomique du BNYVV, ou du cADN correspondant, ce fragment codant pour au moins une partie des protéines codées par les nucléotides 145 à 3285 de la séquence sauvage de l'ARN2, et étant sous le contrôle d'un promoteur permettant l'expression du fragment dans les cellules de la plante et étant dans l'orientation sens ou antisens.

Dans le contexte de l'invention, un fragment "dérivé de l'extrémité 5' de l'ARN2 du BNYVV" signifie le cADN correspondant, ou une variante capable d'hybrider avec celui-ci dans des conditions non stringentes, ou dont le produit d'expression présente au moins 80 % d'homologie. Il est important que les variantes présentent la propriété de pouvoir inhiber l'infection par le BNYVV dans des cellules l'exprimant. Par "inhiber", il faut comprendre une réduction et un retard significatifs de l'apparition des symptômes de l'infection et de la multiplication du virus. Dans des conditions optimales, les symptômes et la multiplication du virus sont éliminés totalement.

Comme exemple de fragment d'acide nucléotidique préféré, on citera un fragment de l'extrémité 5' de l'ARN2 génomique du BNYVV ou du cDNA correspondant, codant pour au moins une partie de la protéine codée par les nucléotides 145 à 2218 du ARN2 ou pour une variante de cette protéine présentant une homologie d'au moins 80 % et comportant l'insertion, la substitution ou la délétion d'acide(s) aminé(s) et qui confère une résistance à la rhizomanie aux cellules l'exprimant.

Un autre exemple d'une plante transgénique selon cet aspect de l'invention est celle dans laquelle ledit fragment code pour la protéine codée par les nucléotides 145 à 708 et, en outre, pour une partie de la protéine codée par les nucléotides 709 à 2218 de l'ARN2 du BNYVV. Une telle plante transgénique peut comporter, selon l'invention, un fragment qui code pour la protéine codée par les nucléotides 145 à 871 de l'ARN2 du BNYVV, ce fragment pouvant être composé, par exemple, par les nucléotides 91 à 871 de l'ARN2 du BNYVV.

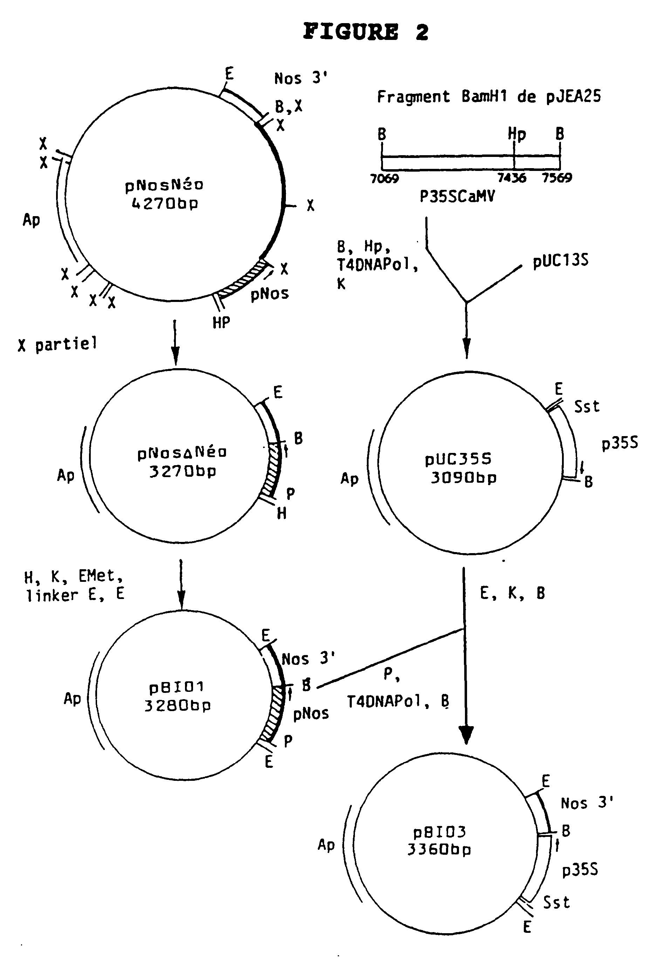

Le fragment transformant peut aussi coder pour au moins une partie d'une variante de la protéine codée par les nucléotides 145 à 2218, ladite variante se distinguant de la séquence sauvage par la présence de la séquence Glu Asp Leu Pro qui remplace les acides aminés His ALa codés par les nucléotides 253 à 258 de la séquence sauvage. Par séquence sauvage, il faut comprendre la séquence de l'ARN2 publiée par Bouzoubaa et al (1986), en particulier celle de la figure 2 de ladite publication. Cette figure indique la séquence nucléotidique ainsi que la séquence d'acides aminés. La numérotation des bases utilisée dans cette demande est la même que celle appliquée par Bouzoubaa et al.

Comme exemples de parties de ce type de variante, on peut citer un fragment qui est composé par les nucléotides 91 à 871 de la séquence sauvage, les nucléotides 253 à 258 de la séquence sauvage étant remplacés par ceux codant pour Glu Asp Leu Pro. La partie de la variante peut aussi correspondre à celle codée par les nucléotides 145 à 255 dans la séquence sauvage, les nucléotides 253 à 255 de la séquence sauvage étant remplacés par ceux codant pour Glu.

Encore un exemple d'une plante transgénique selon cet aspect de l'invention est celle dans laquelle ledit fragment code pour au moins une partie d'une variante de la protéine codée par les nucléotides 145 à 2218, ladite variante se distinguant de la séquence sauvage par la présence de la séquence Arg Ser Ser Gly au lieu des acides aminés codés par les nucléotides 637 à 651 de la séquence sauvage, la séquence Arg Ser Ser Gly formant le carboxy-terminal de la protéine.

Plus particulièrement, les plantes transgéniques, selon ces aspects de l'invention, expriment des fragments d'acide nucléique consistant en les nucléotides 91 à 871 du ARN2 du BNYVV ou de la séquence cADN correspondante, dans lequel les nucléotides 253 à 258 sont éventuellement remplacés par GAA GAT CTT CCT, ou dans lequel la séquence GA AGA TCT TC a été insérée à la position 638, immédiatement après le C en position 637, ou encore les fragments BglII de ces séquences.

Selon un autre mode de réalisation de l'invention, le fragment transformant peut consister en les nucléotides 2078 à 2774 du ARN2 subgénomique du BNYVV. La protéine ainsi exprimée correspond au NH₂ terminal de la protéine de 42kDa du BNYVV.

Des exemples de fragments d'acide nucléique transformants particulièrement préférés et les protéines codées par ces fragments sont illustrés dans le tableau 1 (voir exemple 6).

Parmi ces séquences particulièrement préférées, on citera la séquence de bases consécutives codant pour au moins une partie de la protéine de capside de 22 Kd (codée par les nucléotides 145 à 708) et, en outre, pour une partie de la protéine de 75 Kd (codée par les nucléotides 709 à 2218). Un exemple d'une telle séquence est celle composée des nucléotides 91 à 871 du ARN2. Ce type de séquence incluant le codon stop du 22 Kd donne lieu au phénomène de "readthrough" et la cellule exprime ainsi deux protéines à la fois.

L'invention concerne également les protéines produites par l'expression de ces séquences, et en particulier la protéine de 29 Kd résultant de l'expression des nucléotides 91 à 871 du ARN2, qui est exprimée en même temps que la protéine de capside de 22 Kd.

Les promoteurs qui peuvent être utilisés dans les plantes transgéniques résistantes à la rhizomanie sont tous ceux qui permettent l'expression de la séquence conférant la résistance dans la plante. Les promoteurs 35S et Nos qui sont, respectivement le promoteur du grand transcrit 35S du virus de la mosaïque du choux-fleur et le promoteur du gène de la nopaline synthase sont particulièrement préférés. L'utilisation de ces promoteurs constitutifs, et en particulier le p35S, donne lieu d'une manière surprenante à une expression spécifique de la protéine protectrice dans les racines.

D'autres signaux de transcription à utiliser avec le promoteur sont des terminateurs, par exemple Nos.

Les graines transgéniques des plantes transgéniques résistantes à la rhizomanie font également partie de cet aspect de l'invention.

Les séquences utilisées dans la transformation des plantes de l'invention peuvent être utilisées comme sonde nucléique, en combinaison avec des moyens permettant une détection de l'hybridation, pour détecter la présence des séquences dans des cellules transformées. Ceci peut être utile pour vérifier l'état transformé des cellules.

Cet aspect de l'invention concerne également un procédé de production de plantes transgéniques appartenant à l'espèce Beta vulgaris et résistante à l'infection par le BNYVV, ladite plante exprimant, spécifiquement dans les racines, une protéine capable de conférer ladite résistance, ledit procédé comprenant la transformation, par l'intermédiaire d'Agrobacterium tumefaciens, de cellules provenant de Beta vulgaris avec un des fragments tels que décrits ci-dessus, la transcription dudit fragment étant sous le contrôle d'un promoteur constitutif tel que le p35S ou le pNos, suivi de la régénération d'une plante transgénique à partir des cellules transformées.

Les exemples non limitatifs suivants illustrent notamment :
- la production de fragments de message génétique modifié ou non du virus du BNYVV, par la plante,
- l'expression de ces fragments d'ADN d'origine virale sous des promoteurs constitutifs (35S ; Nos),
- la vérification de cette expression par la mise en évidence des ARN messagers correspondants dans des suspensions cellulaires habituées et transformées.
- la confirmation de la production dans la même cellules de deux protéines de tailles différentes codées par le même gène :
   * la protéine de capside du BNYVV de 22 Kd (188, acides aminés)
   * la protéine chimérique dérivée de la protéine de capside, de 29 Kd (252 acides aminés).
- l'utilisation des trois souches désarmées d'Agrobacterium tumefaciens pour la transformation de suspensions cellulaires et de cals friables de betterave (comparatif),
- l'obtention de suspensions cellulaires transformantes, induites à partir de cals sélectionnés sur kanamycine après transformation de suspensions cellulaires habituées (comparatif),
- l'utilisation de ces suspensions cellulaires transformées comme modèle pour tester l'expression de tout fragment d'ADN placé sous le contrôle de promoteurs appropriés (comparatif),
- l'utilisation de ces suspensions cellulaires pour tester l'inhibition de la multiplication virale par un gène donné (comparatif),
- l'inhibition de la multiplication du BNYVV dans des protoplastes issus de suspensions cellulaires transformées et produisant les deux protéines de 22 Kd et 29 Kd, des protoplastes servant comme modèle pour tester l'infectabilité de la cellule, les cellules ne pouvant pas être infectées directement par les virus (comparatif),
- la transformation de jeunes cals friables organogènes,
- l'obtention de cals transformés organogènes sélectionnés sur kanamycine après transformation de jeunes cals friables organogènes,
- la possibilité de supprimer très tôt à la fois l'agent sélectif (kanamycine) et l'agent bactériostatique (céfotaxime) du milieu de culture des cals,
- le développement des bourgeons ou embryons initiés à partir des cals transformés, en plantes transformées enracinées,
- l'expression dans la plante des gènes transférés,
- la mise en évidence de la production des protéines 22 et 29 Kd spécifiquement dans les racines de betteraves transformées.
- l'absence de la production de protéines 22 et 29 Kd dans la partie aérienne des plantes transformées,
- la production de graines transgéniques à partir de transformants primaires,
- la mise en évidence de la résistance à la rhizomanie des plantules issues des graines transgéniques.

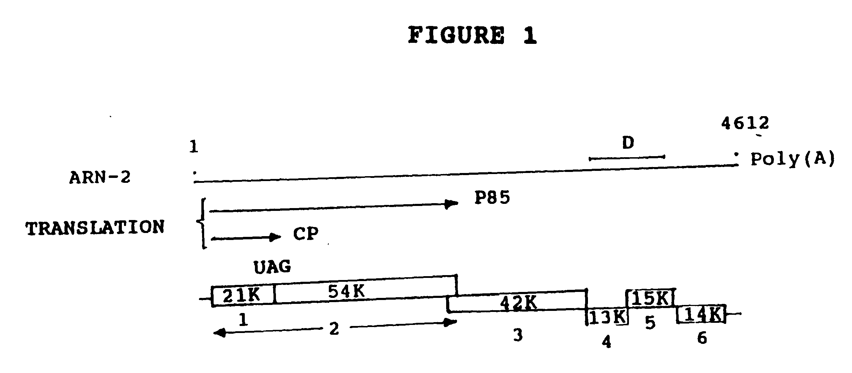

Les figures 1 à 11 et les tableaux 1 à 3 illustrent différents aspects de l'invention, notamment :
**Figure 1** **:**
   Organisation génétique de l'ARN2 du BNYW d'après Bouzoubaa et al, (1986)
**Figure 2** **:** construction des vecteurs d'expression pBIOS1 et pRIOS3.
   pBIOS1 contient le promoteur (pNos) et le terminateur (Nos 3') du gène de la nopaline synthétase séparés par un site de restriction BamHI (B) et encadrés par deux sites EcoRI (E).
   pBIOS3 est un dérivé de pBIOS1 où le promoteur Nos a été remplacé par le promoteur du transcrit 35S (p35S) du Cauliflower Mosaic Virus (CaMV) isolé du plasmide pJEA25 (don de T. Michael).
   Abréviations : B, BamHI - E, EcoRI - H, Hind III - Hp, HphI - P, Pst I - S, Smal - Sst, Sstl - X, XhoII - T4 DNA pol, T4 DNA polymérase - K, klenow polymérase - E Met, EcoRI Méthylase - Ap, Ampicilline - bp, paire de base.

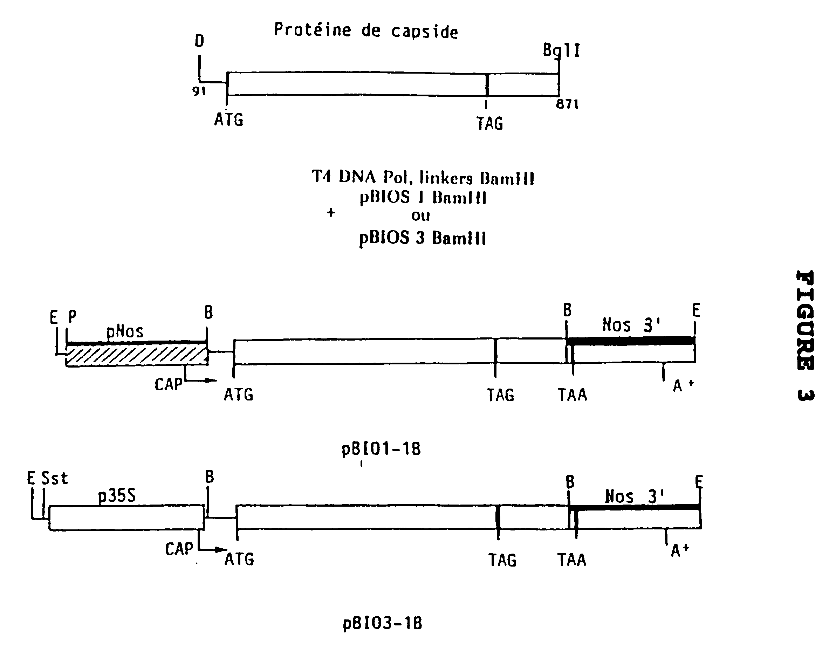

**Figure 3** **:** gènes chimériques codant pour la protéine de capside du BNYW et pour la protéine chimérique dérivée.
   A partir du plasmide pBC2 contenant l'ADN copie de l'extrémité 5' du RNA2, nous avons isolé un fragment Dral-Bgl I de 780 bp (position 91 à 871). Ce fragment a été traité par la T4 DNA polymérase et des linkers BamHI ont été rajoutés permettant son clonage au site BamHI des vecteurs d'expression pBIOS1 et pBIOS3. Les deux plasmides pBIO1-1B et pBIO3-1B obtenus ont le fragment d'ADN en orientation sens.
   Abréviation : B, BamHI - D, Dra I - E, EcoRI - p, PstI - Sst, Sst 1 - T4 DNA Pol, T4 DNA Polymérase - CAP, départ théorique du transcrit - A+, signal de polyadénylation - ATG, codon de départ - TGA, TAA, codons stop.
**Figure 4** **:** construction de pBIO1-2B et pBIO1-5B.
**Figure 5** **:** construction des vecteurs de transfert.
   Les différentes entités génétiques fonctionnelles dérivées de pBIOS1 et de pBIOS3 sont insérées dans le vecteur binaire pGA492. Les dérivés de pBIOS1 sont clonés au site EcoRI et seuls sont retenus les construits orientés dans le sens de transcription identique au gène de la néomycine phophotransférase (npt), alors que les dérivés de pBIOS3 sont insérés entre le site Sstl et le site EcoRI, et se trouvent obligatoirement dans la bonne orientation. 32 plasmides dérivés de pGA492 ont donc été obtenus.
   Abréviations : E, EcoRI - Sst, Sstl - npt, néomycine phosphotransférase - cat, chloroamphénicol acétyltransférase - BR et BL, bordures droite et gauche du T-DNA - Tet, tétracycline résistance - Kb, kilobase.

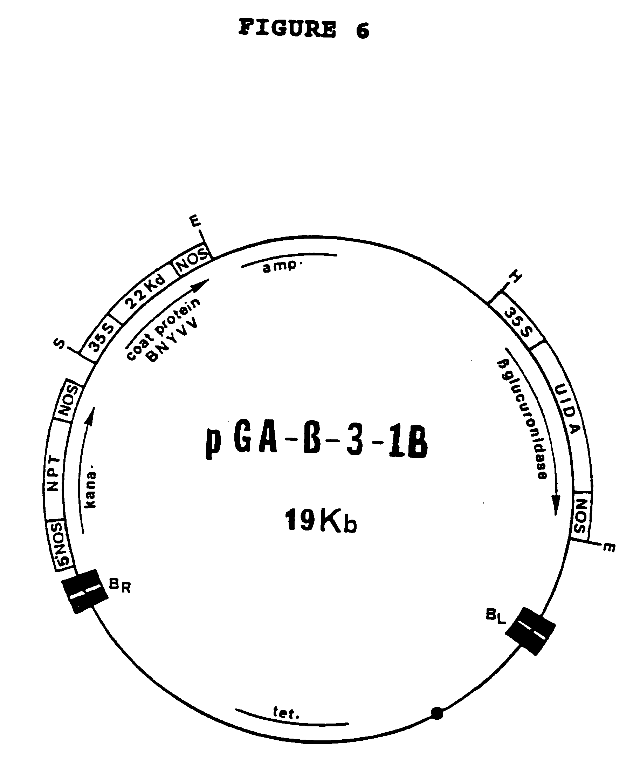

**Figure 6** : vecteur binaire pGA-β -3-1 B.
   Légende : 35S, promoteur 35S du Cauliflower Mosaic Virus - 5'Nos, promoteur du gène de la nopaline synthase - Nos, terminateur du gène de la nopaline synthase. NPT, gène de la néomycine phosphotransférase -UID A, gène de la -glucuronidase d'E. coli - BR et BL, bordures droite et gauche du T-DNA de pTiT 37 - amp, gène de résistance à l'ampicilline - tet., gène de résistance à la tetracycline - Kana., gène de résistance à la kanamycine - E, EcoRI - H, Hind III - S, Sst I - ● ,origine de réplication du RK2.

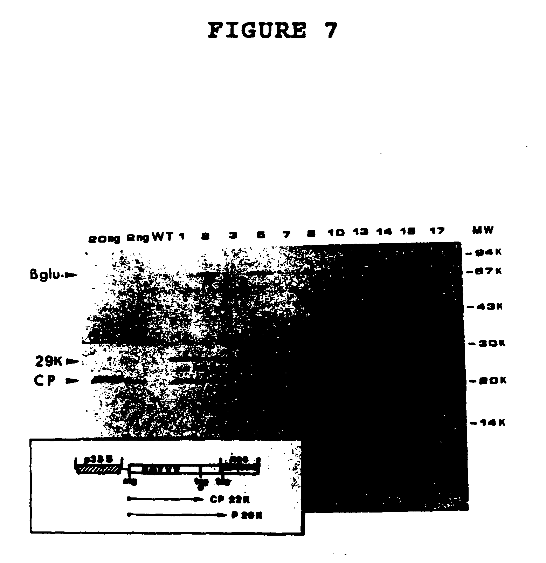

**Figure 7** : analyse par Western blot des suspensions cellulaires transformées par le vecteur binaire pGA- β-3-1B.
   Légende : CP, protéine de capside - WT, suspension non transformée - 20 ng et 2 ng, reconstructions avec les quantités indiquées de BNYW β-Glu, β-glucuronidase - MW, marqueur de poids moléculaire.
   En encadré, la structure du gène chimérique codant pour la protéine de capside est représenté. p35S, promoteur du Cauliflower Mosaic Virus - Nos, terminateur du gène de la nopaline synthase ; tag, codon de terminaison du BNYVV : readthrough - tag, codon de terminaison situé dans le terminateur - atg, codon d'initiation.

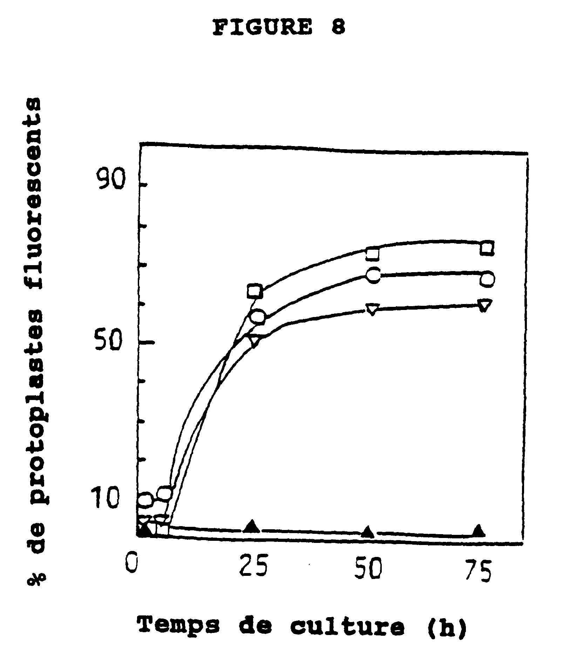

**Figure 8** **:** infection de protoplastes de betterave par du BNYVV F13 (□,○) et S2 (∇) par la méthode au PEG. (▲) représente les résultats obtenus avec du virus inactivé.

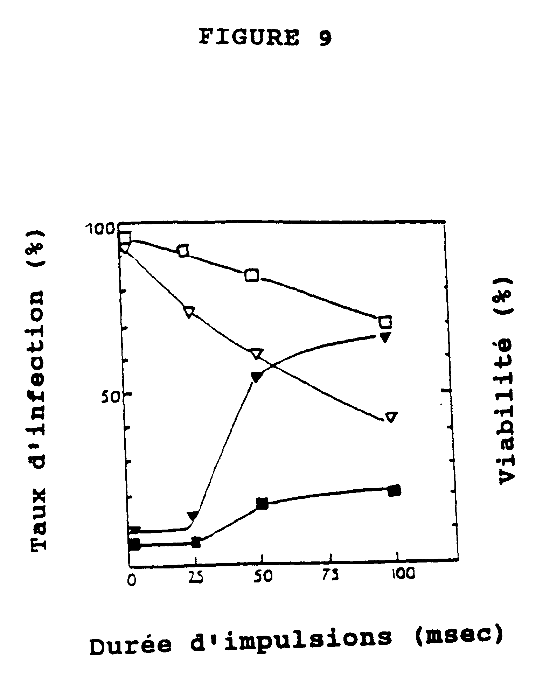

**Figure 9** **:** infection de protoplastes de betterave par électroporation en présence ( ) et en absence (△) de CaCl2 5 mM. Les symboles ouverts correspondants représentent le pourcentage de protoplastes viables en présence (∇) et en absence (□) de CaCl2 5 mM.

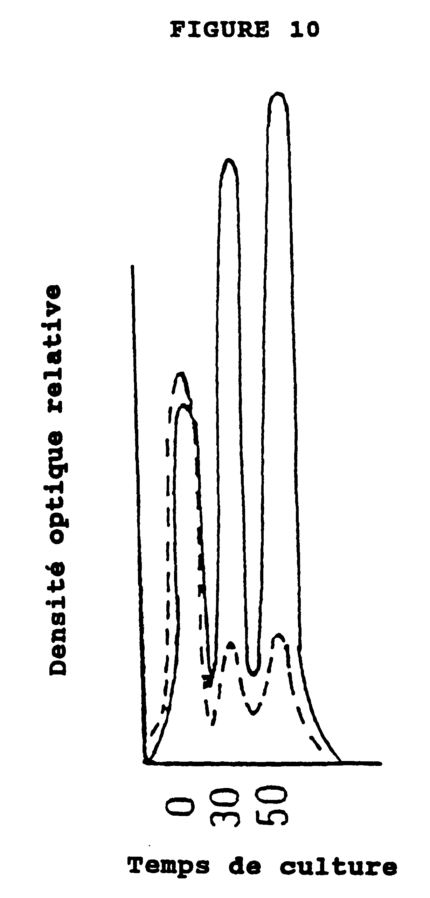

**Figure 10** **:** profils densitométriques d'analyses de RNA extraits de protoplastes infectés isolés de lignées exprimant (----) ou n'exprimant pas (--) la protéine de capside du BNYVV.

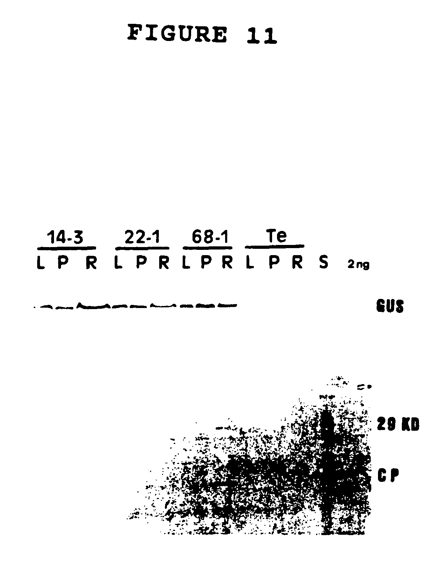

**Figure 11** **:** analyse par Western blot d'extraits protéiques de 3 betteraves transgéniques à l'aide de deux sérums : un sérum anti-BNYVV et un sérum anti-β -glucuronidase.
   Abréviations : L, limbe ; P, pétiole ; R, racine ; Te, témoin ; S, suspension cellulaire transformées ; 2ng, 2ng de BNYVV purifié ; 14-3, 22-1 et 68-1 réfèrent à 3 betteraves transgéniques différentes.

**Tableau 1 :** les 16 gènes de résistance potentiels au BNYVV avec leurs produits théoriques.

N.B. : les chiffres entre parenthèses réfèrent la position des sites de restrictions selon la séquence du BNYVV ; △ représente le linker BglII additionné. La taille des transcripts correspond à la partie du messager complémentaire du BNYVV.

**Tableau 2 :** infection par le BNYVV de protoplastes issus de cellules transformées et non transformées. 1 x 10⁶ protoplastes ont été inoculés avec 5 µg de BNYVV (F13 ou S2) sauf pour l'expérience 4 où l'inoculum consistait de 30 µg de BNYVV les chiffres représentent le pourcentage de protoplastes fluorescents détectés 30 h après l'inoculation. CP+ et CP- sont des lignées cellulaires transformées exprimant et n'exprimant pas la protéine capsidaire du BNYVV.

**Tableau 3 :** différents milieux de culture utilisés dans le procédé de transformation et de régénération selon l'invention.

### EXEMPLE 1 : EXTRACTION DE L'ARN VIRAL

La multiplication du virus BNYVV se fait sur Chenopodium quinoa après inoculation manuelle, et l'extraction se fait à partir de feuilles virosées de huit jours selon la technique de Putz (1977). La suspension virale ainsi obtenue est ajustée à 100 mM NaCl puis soumise à deux extractions au phénol suivies de deux lavages à l'éther de la phase aqueuse. Les RNA viraux sont précipités par addition de deux volumes d'éthanol distillé et conservés à -20 ° C. Le nombre et la taille des RNA a été rapporté par Richards et al (1985).

### EXEMPLE 2 : FABRICATION DU cDNA DU RNA 2

Richards et al ont montré en 1985 que le RNA 2 était un messager efficace pour la synthèse de la protéine capsidaire. Un ADN complémentaire d'une partie du RNA 2 a été synthétisé par la méthode de Van der Werf et al (1981) et cloné au site Pst 1 du plasmide pBR322 (Bolivar et al, 1977). Le plasmide résultant pBC2 contient un ADN copie correspondant aux 2938 bases de l'extrémité 5' du RNA 2 (Richards et al, 1985).

### EXEMPLE 3 : DETERMINATION DE LA SEQUENCE DE L'EXTREMITE 5'DU RNA 2

Grâce à la technique de Maxam et Gilbert (1980), et à celle de Sanger (1977), la séquence du clone pBC2 a été déterminée. La séquence détaillée est présentée dans la publication de Bouzoubaa et al (1986) avec l'organisation génétique du RNA2 schématisée. L'examen de cette séquence a confirmé la présence de la protéine de capside au niveau du 1er cistron situé en 5', le codon ATG de départ étant placé à 144 nucléotides de l'extrémité 5'. Cette protéine d'un poids moléculaire de 22 Kd comporte 188 codons et se termine par un codon ambre UAG. La composition en acides aminés déduite de la séquence est identique à celle de la protéine capsidaire du BNYV\/ déterminée par C. Putz (1977). Le codon stop (UAG) est immédiatement suivi d'une phase ouverte ayant une capacité codante correspondant à un polypeptide de 54 Kd. Par la suppression du codon UAG, on peut donc obtenir une phase de lecture ouverte capable de coder pour une protéine de 75 Kd. Ces données sont en accord avec les résultats obtenus par Ziegler et al (1985) montrant que le RNA2 est un messager efficace pour la synthèse de deux protéines immunoprécipitées par du sérum anti BNYVV et dont la synthèse est augmentée en présence d'un t.RNA suppresseur. Tous ces résultats sont discutés dans la publication de Bouzoubaa et al (1986), il est aussi précisé qu'une 3ème phase de lecture capable de coder in vitro pour une protéine se fait à partir d'un ARN subgénomique du RNA2 qui a été mis en évidence et qui peut être encapsidé.

### EXEMPLE 4 : CONSTRUCTION DES VECTEURS D'EXPRESSION

Premièrement les signaux de transcription du gène de la nopaline synthase de la bactérie Agrobacterium tumefaciens T 37 ont été utilisés. A partir du plasmide pNopnéoΔ 18 construit par Bevan (1983) dont la structure est présentée dans la figure 2, celui-ci contient la séquence du vecteur de clonage pUC9 (Vierra et al, 1982) avec un fragment EcoRI-BamHI de 260 bp et un fragment HindIII-XhoIII (BamHI-BglI) de 310 bp. Ces deux fragments qui proviennent du plasmide tumorigène pTiT37 d'Agrobacterium tumefaciens T 37 contiennent respectivement le signal de polyadénylation et le promoteur du gène de la nopaline synthase. Ces deux signaux de transcription encadrent un fragment BglII-Bam HI de 1000 bp qui contient le gène codant pour l'aminoglycoside phosphotransférase II ; à partir de ce plasmide, le fragment de 1000 bp a été enlevé par digestion ménagée avec l'enzyme de restriction Xholl, et les molécules de taille de 3300 bp environ sont récupérées après migration sur gel d'agarose. Après ligation et transformation dans E. coli HB101 (Bolivar et al, 1979) des transformants résistants à l'ampicilline ont été sélectionnés. La majorité de ces techniques sont décrites dans "Molecular Cloning. A laboratory manual" (Maniatis et al, 1982). Grâce à la cartographie à l'aide d'enzyme de restriction, les inventeurs ont retenu le plasmide pNosΔNéo, qui possédait un fragment EcoRl-BamHl de 260 bp et un fragment BamHI-HindIII de 310 bp. Pour des commodités d'utilisation de ce plasmide, les inventeurs ont remplacé le site de restriction Hind III par un site EcoRI (détails non présentés) et ainsi ils ont obtenu le vecteur d'expression pBIOS1 (figure 2).

Un autre vecteur d'expression a été construit dans le cadre de l'invention. Il a la même structure que pBIOS1, le même fragment comportant le signal de polyadénylation, mais le fragment promoteur a été changé. En effet, le fragment PstI-BamHI comportant le promoteur du gène de la nopaline synthase a été remplacé par un fragment EcoRI-BamHI de 400 bp contenant le promoteur du grand transcrit 35S du virus de la mosaïque du choufleur (Ca.M.V.). Ce fragment a été obtenu à partir du plasmide pUC35S qui dérivait du clonage d'un fragment BamHI-HphI au site Sma I du vecteur de clonage pUC13 (Messing, 1983). La source du fragment BamHI-Hphl était le plasmide pJEA25 construit par T. Michael qui avait prélevé un fragment Dde I s'étendant de la position 7069 à la position 7569 du CaMV isolat BJ1 décrit par Franck et al (1980). Après avoir modifié les extrémités de ce fragment grâce à des linkers BamHI, T. Michael l'avait cloné au site BamHI du plasmide pAT153 (Twigg et al, 1980). Sur la figure 2, les différentes étapes de la construction du vecteur d'expression pBIOS3 sont schématisées. Pour les deux vecteurs d'expression pBIOS1 et pBIOS3, le site de restriction BamHI est présent entre les deux fragments d'ADN contenant le promoteur pour l'un et pour l'autre le signal de polyadénylation ; cette situation est idéale pour l'insertion de tout DNA étranger qui sera un substrat pour la transcription.

### EXEMPLE 5 : INSERTION DE FRAGMENTS cDNA DU BNYVV AU SITE BAMHI DES VECTEURS D'EXPRESSION

Le plasmide pBC2 a été digéré par les enzymes de restriction BglI et Dral et ensuite l'extrémité sortante du site BglI a été supprimée par l'utilisation de la DNA polymérase du bactériophage T4. Ce fragment a été purifié par élution à partir d'un gel d'agarose, après séparation électrophorétique. Des linkers BamHI ont été additionnés aux extrémités de ce fragment, et après une digestion avec un grand excès de l'enzyme de restriction BamHI ; ce fragment de 780 bp a été incubé avec les vecteurs pBIOS1 et pBIOS3 ouvert au site BamHI. Après ligation, le mélange a servi à transformer E. coli HB101. Après un tri effectué sur les clones résistants à l'ampicilline, 4 plasmides ont été retenus. Les plasmide appelés pBIO1-1B et pBIO3-1B qui ont le fragment cDNA contenant la séquence codante de la protéine de capside sous le contrôle du promoteur Nos pour le premier et pour l'autre sous le contrôle du 35S (figure 3).

Ces plasmides peuvent diriger la fabrication de RNA messagers qui une fois traduits, produiront à la fois la protéine de capside du BNYVV et une protéine chimérique de 29 Kd provenant de la suppression du codon stop (phénomène de readthrough). Cette protéine de 252 acides aminés est composée comme suit :
- les 188 acides aminés de la protéine de capside,
- 53 acides aminés de la protéine de 75Kd allant de la position 189 à 241 inclus,
- de 11 acides aminés codés par la séquence du terminateur de la nopaline synthase qui sont respectivement : thréonine, glycine, serine, proline, isoleucine, leucine, glutamine, thréonine, phénylalanine, glycine, glutamine. Deux autres plasmides ont été obtenus, ces plasmides appelés pBIO1-1A et pBI03-1A ont le fragment cDNA du BNYVV en orientation inverse sous le contrôle des deux promoteurs. Ces plasmides pourront diriger la fabrication de RNA messagers dits antisens, qui seront des messagers complémentaires de la partie 5' du RNA2.

### EXEMPLE 6 : CONSTRUCTION D'UNE FAMILLE DE GENES PERMETTANT LA FABRICATION DE PROTEINES DE CAPSIDE DU BNYVV MODIFIEES ET DE RNA ANTISENS DE TAILLE VARIABLE

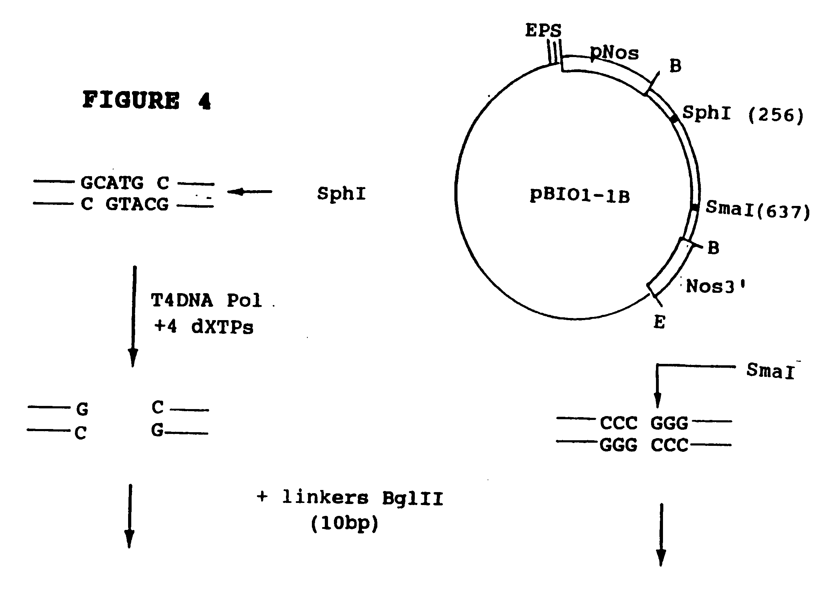

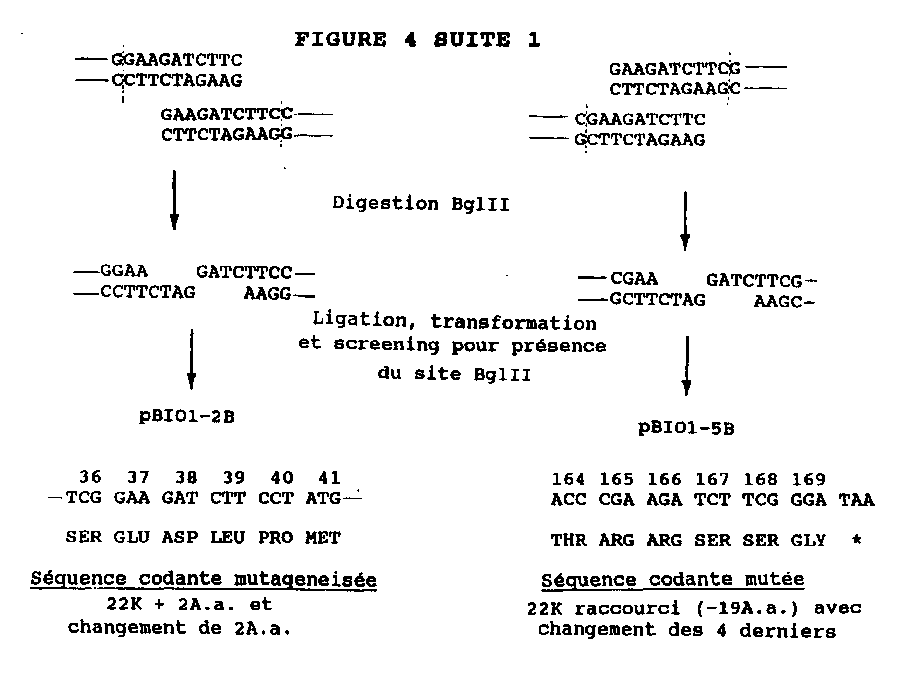

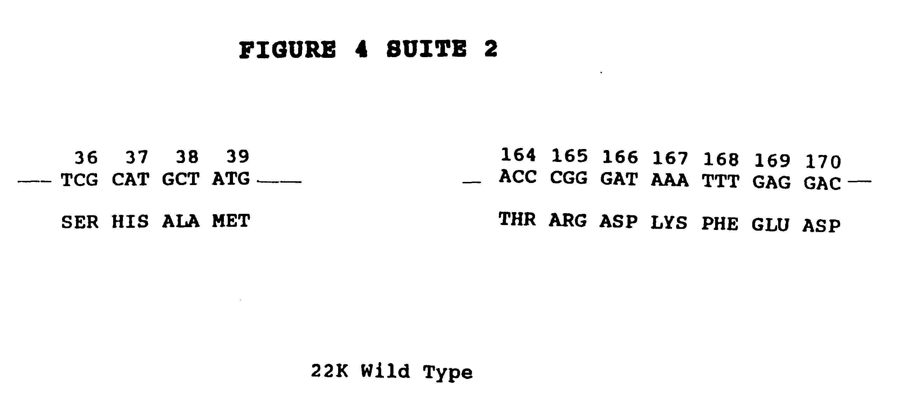

Deux plasmides dérivés de pBIO1-1B, le plasmide pBIO1-2B et le plasmide pBIO1-5B ont été construits. Les différentes étapes de la construction de ces plasmides sont présentées dans la figure 4. Brièvement pour la construction de pBIO1-2B, le plasmide pBIO1-1B a été ouvert au site SphI (position 256 du RNA2) les bouts "collants" ont été enlevés par l'utilisation de la DNA polymérase du bactériophage T4 (délétion de 4 bp) et des linkers BglII de 10 bp ont été ligasés aux extrémités franches ainsi produites. Après une digestion exhaustive avec BglII, le plasmide est religasé. Après transformation dans E. coli, le plasmide pBIO1-2B a été obtenu ; celui-ci ne contient plus de site SphI et à cette position on trouve maintenant un site BglII. Cette manipulation a permis deux choses : premièrement, l'obtention d'un site de restriction utile pour d'autres constructions (extrémités compatibles avec celles produites par BamHI) et deuxièmement, l'obtention d'une séquence codante modifiée qui code pour une protéine de capside possédant deux acides aminés supplémentaires et dont deux acides aminés sont changés. Le plasmide pBI01-5B a été obtenu par le même type de manipulation mais dans son cas c'est le site Smal de pBIO1-1B (position 637 du RNA2) qui a été modifié par des linkers BglII. Ce plasmide code pour une protéine de capside raccourcie de 19 acides aminés et dont les 4 derniers sont modifiés.

En utilisant les sites BglII et Bam HI de ces deux plasmides, les inventeurs ont pu isoler et cloner dans les deux vecteurs d'expression pBIOS1 et pBIOS3 différents morceaux de cDNA recouvrant l'extrémité 3' ou l'extrémité 5' de la protéine de capside et ceci sur des longueurs variables. Une famille de gènes susceptibles de rendre résistante au BNYVV une cellule ou une plante fabriquant le produit d'un ou plusieurs de ces gènes a ainsi été obtenue. Tous ces différents fragments de cDNA sont présentés dans le tableau 1 avec les produits attendus (transcrit et protéine) ; la lettre B référant une orientation sens et A une orientation antisens.

**TABLEAU 1**

| CODE | FRAGMENT cDNA | TRANSCRIPT | PROTEINE |
|---|---|---|---|
| 1B | 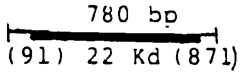 | ARN bon sens | protéine de capside de 22 Kd (188 acides aminés) et protéine chimérique dérivée 29 Kd (252 Aa) |
| | | 780 bp | |
| 2B | 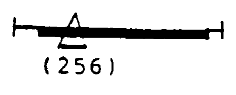 | ARN bon sens | Protéine de capside mutée (190 Aa) |
| | | 780 bp | |
| 3B | 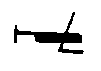 | ARN bon sens 3' | 36 Aa NH2 terminal de la protéine de capside |
| | | 165 bp | |
| 4B | 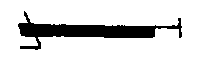 | ARN bon sens 5' | |
| | | 615 bp | |
| 5B | 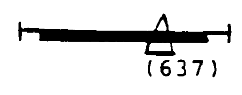 | ARN bon sens | Protéine de capside tronquée (169 Aa) |
| | | 780 bp | |
| 6B | 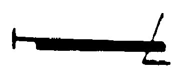 | ARN bon sens 5' | 164 Aa NH2 terminal de la protéine de capside |
| | | 546 bp | |
| 7B | 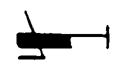 | ARN bon sens 3' | |
| | | 234 bp | |
| 8B | 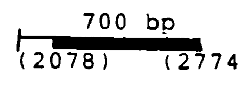 | ARN bon sens | 215 Aa NH2 terminal de la protéine de 42 Kd |
| | | 700 bp | |
| 1 A | 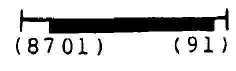 | ARN anti-sens | |
| | | 780 bp | |
| 2 A | 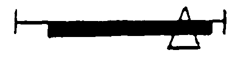 | ARN anti-sens | |
| | | 780 bp | |
| 3 A | 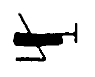 | ARN anti-sens | |
| | | 165 bp | |
| 4 A | 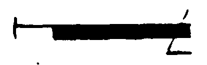 | ARN anti-sens | |
| | | 3' 615 bp | |
| 5 A | 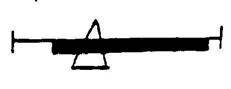 | ARN anti-sens | |
| | | 780 bp | |
| 6 A | 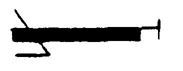 | ARN anti-sens | |
| | | 5' 546 bp | |
| 7 A | 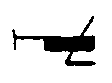 | ARN anti-sens | |
| | | 3' 234 bp | |
| 8 A | 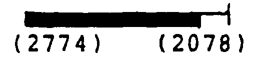 | ARN anti-sens | |
| | | 700 bp | |

| | | | |
|---|---|---|---|
| N.B. : les chiffres entre parenthèses repèrent la position des sites de restrictions selon la séquence du BNYVV ; ∇ représente le linker BglII additionné. La taille des transcripts correspond à la partie du messager complémentaire du BNYVV. | | | |

Sur ce tableau, il est aussi présenté un fragment de cDNA s'étendant de la position 2078 à 2774 ; il s'agit d'un fragment Sau 3A du RNA2 correspondant à l'extrémité 5'du RNA subgénomique codant pour la protéine de 42 Kd (figure 1b). Les inventeurs ont cloné ce fragment dans les vecteurs d'expression pBIOS1 et pBIOS3 et ceci dans les deux orientations (8B et 8A).

### EXEMPLE 7 : INTRODUCTION DES GENES DANS UN VECTEUR BINAIRE

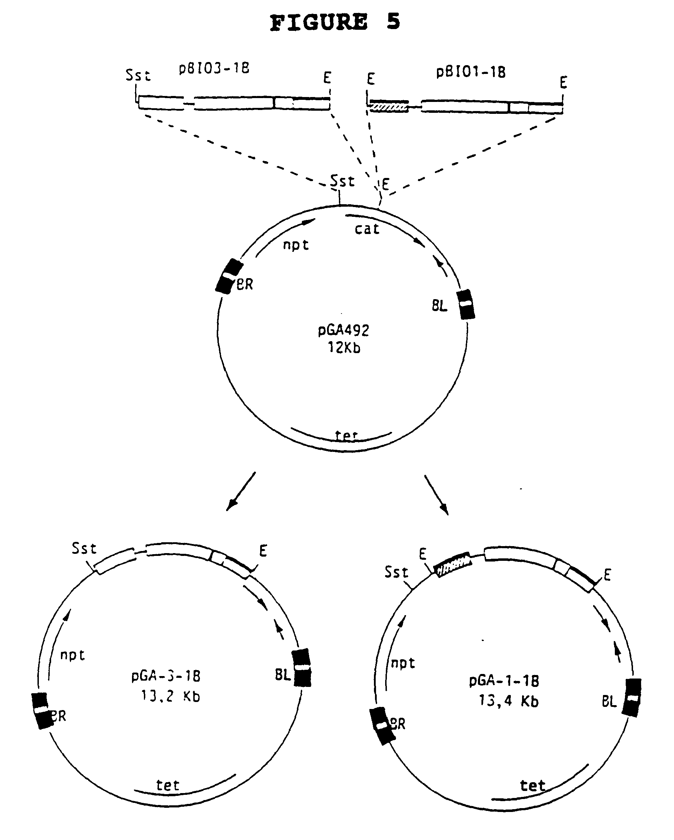

Le vecteur binaire pGA492 choisi a été construit par G. An (1986).Ce plasmide a une taille de 12 kb, sa carte génétique est présentée dans la figure 5. Ce plasmide possède :
- deux fragments d'ADN contenant les séquences du T-DNA du plasmide tumorigène pTiT37, ces séquences délimitent la partie transférée et sont indispensables au transfert,
- un gène chimérique contenant la séquence codante du gène de la néomycine phosphotransférase du transposon Tn5 (Rothstein et al, 1981) qui est fusionné à un fragment contenant le promoteur du gène de la nopaline synthase et les premiers codons de la séquence codante de ce même gène. Ce gène chimérique est terminé par le signal de polyadenylation du gène de la nopaline synthase. Ce gène confère aux cellules végétales le contenant la capacité de se multiplier en présence de kanamycine, qui est normalement un antibiotique toxique pour celles-ci,
- plusieurs sites uniques de restriction permettant le clonage de gènes construits in vitro,
- une origine de réplication à large spectre d'hôte fonctionnant dans E. coli et dans Agrobacterium tumefaciens,
- une origine de transfert et les gènes mob nécessaires pour le transfert par conjugaison bactérienne,
- un gène de résistance à la tétracycline qui permet la sélection de bactéries transconjugates,
- un gène de résistance à la tétracycline qui permet la sélection de bactéries transconjugantes.

Sur la figure 5, les inventeurs ont exemplifié l'introduction de deux gènes, les gènes portés par pBIO1-1B et par pBIO3-1B. Tous les fragments de cDNA sous le contrôle du promoteur Nos (pBIOS1 dérivés) sont clonés au site EcoRI de pGA 492 après isolement des gènes respectifs avec EcoRI. Seuls les clones présentant les gènes dans le même sens de transcription que celui du gène conférant la résistance à la kanamycine sont retenus. Pour les gènes contenant le promoteur 35S du CaMV (pBIOS3 dérivés) le clonage s'effectue entre les sites Sst I et EcoRI de pGA 492 après digestion des diférents plasmides par ces deux mêmes enzymes. 32 vecteurs de tranfert ont été obtenus, deux de ceux-ci sont présentés dans la figure 5, pGA-1-1B et pGA-3-1 B.

### EXEMPLE 8 : INTRODUCTION D'UN GENE REPORTEUR DANS LE VECTEUR BINAIRE pGA-3-1B

La confirmation du caractère transformé d'un tissu végétal s'effectue dans de nombreux cas par la mise en évidence des protéines codées par les gènes transférés. Dans le cas du vecteur binaire pGA-3-1B, les inventeurs ont pu doser par des techniques immunologiques la protéine de capside du BNYVV et par un dosage enzymatique l'activité de la néomycine phosphotransférase qui confère la résistance à la kanamycine. Cependant, ces dosages sont longs et délicats et ils nécessitent une importante quantité de matériel végétal.
En 1987, Jefferson et al, utilisent le gène uid A d'E. coli codant pour l'enzyme β-glucuronidase (GUS E.C.3.2.2.31) comme gène reporteur. Ce gène sous le contrôle de signaux de transcription végétaux (promoteur 35S du CaMV et terminateur Nos) s'exprime très bien dans les cellules végétales et l'enzyme se révèle très stable. Plusieurs méthodes de dosage de l'activité enzymatique de cette protéine sont disponibles car il existe différents types de substrats ; en particulier des substrats donnant des produits chromogènes et des substrats donnant des produits fluorescents. On peut effectuer de façon simple et avec peu de matériel végétal des dosages quantitatifs (intensité de la fluorescence) et des dosages qualitatifs par histochimie (apparition d'un précipité bleu-indigo). Pour bénéficier de ce système reporteur dans lés expériences de transformation, les inventeurs ont construit le vecteur binaire pGA-β-3-1B. La structure génique de ce plasmide est présentée dans la figure 6. Le gène pouvant inhiber la multiplication du BNYVV est encadré par le gène conférant la résistance à la kanamycine et par le gène codant pour la β-glucuronidase. Pour réaliser cette construction, les inventeurs ont inséré au site EcoRI du vecteur binaire pGA-3-1B, le plasmide pB1221 (Jefferson, 1987) ouvert à sont site EcoRI. Le vecteur binaire pGA-β-3-1B résultant (d'une taille de 19 kb) a été transféré dans les souches d'Agrobacterium désarmées (LBA 4404, EHA 101, C58'3) par conjugaison triparentale selon la technique décrite par Ditta et al (1980).

### EXEMPLE 9 : OBTENTION DE CALS FRIABLES REGENERANTS A PARTIR DE FEUILLES

Des graines de betterave sont semées dans du terreau en serre. Ces graines sont issues de la variété américaine "REL 1 ".

Environ un mois après la germination en serre, les premières expériences d'induction de cals selon la méthode décrite par Saunders et al (1986) ont été faites :
- de jeunes feuilles de 3 à 5 cm de long sont prélevées de chaque plante,
- elles sont désinfectées comme suit :
   détergent de la marque Domestos 15 % pendant 5 minutes
   3 rinçages a l'eau stérile
   séchage sur papier filtre stérile
- chaque feuille est ensuite découpée en fragments de 0.25 cm² environ,
- les explants ainsi obtenus sont mis en culture sur du milieu MSB1 (Tableau 3) en boîtes de Pétri,
- les boîtes après avoir été scellées avec un film plastique de la marque Scello-frais sont placées à 30 °C à l'obscurité pendant 30 jours,
- puis elles sont sorties en chambre de culture L:D 18:8, 25 ° C:20 ° C.

De 4 à 10 semaines après la mise en culture, des cals blancs friables apparaissent autour, sur ou sous les explants foliaires.

De 1 à 8 semaines après l'apparition de cals des bourgeons et/ou embryons commencent à régénérer de ces cals.

**TABLEAU 3 : MILIEU DE CULTURE**

| MS : | |
|---|---|
| Pour 1 litre : | |
| | 4.4 g de milieu de Murashige et Skoog |
| | déshydratés de chez SIGMA réf. M6899. |

| Vitamines : | |
|---|---|
| | 1 mg panthoténate de calcium |
| | 0,01 mg biotine |
| | 1 mg acide nicotinique |
| | 1 mg pyridoxine HCl |
| | 10 mg thiamine HCl |
| 30 g saccharose | |
| pH : 5.8 | |
| Milieu solide : 8 g agar-agar | |
| | |

| MSB1 : | |
|---|---|
| MS + 1 mg/l BAP | |
| | |

| MSBo.1 : | |
|---|---|
| MS + 0.1 mg/l BAP | |

| MS enracinement : | |
|---|---|
| MS + 1 mg/l ANA | |

| Etalement des cellules : | |
|---|---|
| | NSB1 + C = MSB + 300 mg/l céfotaxime |
| | MSB1 + CK = MSB + 300 mg/l céfotaxime + 200 mg/l |
| | kanamycine |
| | |

| LB : | |
|---|---|
| Pour 1 litre : | |
| | 10 g bactotryptone |
| | 5 g yeast extract |
| | 10 g MaCl |

### EXEMPLE 10 : OBTENTION DE SUSPENSIONS CELLULAIRES A PARTIR DES CALS INDUITS (COMPARATIF)

De 4 à 6 semaines après leur apparition, les cals sont prélevés (en évitant toute structure organisée) et mis en culture dans 100 ml de milieu MSB1 liquide, dans les erlenmeyers de 250 ml fermés avec une feuille de cellophane maintenue au col par deux élastiques. Les erlenmeyers sont agités à 200 RPM environ dans la chambre de culture.

La suspension cellulaire s'établit en 2 ou 3 semaines. Chaque suspension est repiquée toutes les 3 semaines environ comme suit :
- le contenu de chaque erlenmeyer après 3 semaines de culture est filtré sur une série de trois tamis empilés (dont les mailles sont de 1 mm, 500 µm et 100 µm). Une partie de chaque fraction (>1 mm, >500µm, >100 µm) est remise en suspension dans 100 ml de milieu MSB1 frais en erlenmeyers de 250 ml. Ces nouvelles suspensions sont à nouveau agitées à 200 RPM. L'observation des différentes suspensions cellulaires établies à partir des différents génotypes ont permis de distinguer deux types cellulaires :
   * type habitué (A) : suspension fine, verte à croissance rapide, régénère ponctuellement des formations vitrifiées se développant difficilement.
   * type noduleux (C) : suspension d'agrégats compacts jaunâtres, à croissance plus lente, régénère plus fréquemment que la première, des structures embryonnaires compactes se développant assez bien.

### EXEMPLE 11 : TRANSFORMATION DE SUSPENSIONS CELLULAIRES ET DE DISPERSIONS DE JEUNES CALS

### Suspension cellulaire (comparatif)

La transformation est faite sur des suspensions cellulaires après 3 semaines de culture, sans repiquage.

Dans un tube plastique, stérile et gradué, on recueille une partie de la suspension de manière à avoir 5 ml de cellules tassées dans 10 ml de milieu. 10 ml milieu MSB1 frais sont rajoutés. La nouvelle suspension ainsi obtenue est distribuée dans quatre boîtes de pétri à raison de 5 ml par boîte.

Les souches d'Agrobacterium tumefaciens testées sont LBA 4404, EHA 101, C58'3. Chacune de ces souches contient des vecteurs binaires portant les gènes construits (exemples 5 et 6) et plus particulièrement le vecteur pGA-β-3-1B (exemple 8).

Les souches bactériennes sont conservées à -20ºC dans 15 % de glycérol. 50 µl de chaque souche sont prélevés et mis en culture dans 2 ml de milieu LB + rifampicine + tetracycline. Les cultures sont agitées à 200 RPM à 30ºC pendant 2 jours. Chaque souche est repiquée dans du milieu frais et cultivée dans les conditions décrites plus haut pendant une nuit.

Quand les bactéries sont ainsi prêtes, l'infection des cellules végétales est faite :
- 50 µl de chaque souche poussée une nuit sont prélevés et ajoutés à une des boîtes de Pétri contenant les cellules de betteraves décrites plus haut
- la coculture des cellules de betterave et des bactéries se fait à l'obscurité pendant 3 jours en chambre de culture
   Après ces 3 jours, des cellules végétales sont lavées pour éliminer la bactérie, une première fois avec du MSB1 + 600 mg/l de céfotaxime (bactériostatique inhibant la croissance d'Agrobacterium), puis une deuxième fois dans du milieu MSB1 + 300 mg de céfotaxime
- les cellules ainsi lavées sont mises en culture sur un disque de papier Whatman stérile déposé sur du milieu MSB1 + CK (tableau 3), en boîtes de Pétri (la kanamycine est l'agent sélectif permettant aux seules cellules transformées de se développer). Les boîtes sont scellées avec du scello-frais et mises en chambre de culture, 15 jours après, les filtres portant les cellules végétales sont repiqués sur du milieu frais M8B1 + céfotaxime + kanamycine
- 3 à 8 semaines après la coculture, des cals blancs apparaissent sur un lit de cellules mortes.

Quand ils sont suffisamment développés, ces cals sont repiqués soit sur milieu MSB1 + CK ou MSB1 + C ; la plupart des cals poussent sur les deux milieux. Un test histochimique (Jefferson. 1987) pour déceler l'activité de la protéine codée par le gène de la β-glucuronidase dans les cellules de ces cals, a permis d'évaluer à environ 80 % le taux de cals positifs pour ce test. Ceci confirme donc le caractère transgénique de la plupart des cals obtenus.

Le fait de cultiver ces cals sans l'agent sélectif (kanamycine) ne semble pas modifier l'expression du gène GUS. De plus, après un mois de culture sur milieu avec céfotaxime, les cals peuvent être sevrés de cet antibiotique sans que la bactérie se développe sur le milieu.

Donc, un mois après le clonage des cals, on peut se passer d'ajouter les deux antibiotiques dans le milieu de culture, sans inconvénient apparent. Ceci peut représenter un atout pour la régénération.

### Dispersion de cals nouvellement induits

Au lieu de transformer des suspensions cellulaires induites depuis plusieurs mois (et ayant hypothétiquement perdu de leur potentiel de régénération), de jeunes cals fraîchement induits d'explants foliaires (Saunders et al, 1988) étaient transformés. Le potentiel organogène de ces cals transformés pouvait ainsi être investigué.

Pour tester cela, des cals juste apparus depuis 2 à 8 semaines sur feuilles de serre ont été prélevés. Ces cals ont été dispersés dans un milieu MSB1 liquide en tubes plastiques stériles, et le même protocole de transformation que pour les suspensions cellulaires a été appliqué. Des cals transformés ont ainsi été sélectionnés par cette voie.

### EXEMPLE 12 : EXPRESSION DES GENES POUVANT INHIBER LE DEVELOPPEMENT DU BNYVV DANS DES CELLULES DE BETTERAVE (COMPARATIF)

Les inventeurs ont transformé des suspensions cellulaires habituées de betteraves avec tous les gènes construits présentés dans les exemples 5 et 6. Pour chacun de ces gènes plusieurs suspensions cellulaires transformées ont été initiées et cultivées en présence de kanamycine 200 mg/l. A partir de 10 g de cellules transformées soumises à l'action de cellulases et de pectinases, soit les ARN totaux, soit les protéines solubles totales ont été isolés.

Pour l'extraction des ARNs la technique utilisant l'isothyocyanate de guanidium décrite par Ausubel et al (1987) s'est révélée la plus efficace sur ces cellules aux parois digérées. Les ARN totaux ont été analysés par Northern blot selon Maniatis et al (1982). Pour l'hybridation des ARN totaux contenant un ARN message dérivé du gène de la protéine de capside, les inventeurs ont utilisé comme sonde le fragment BamHI de 780 paires de bases du plasmide pRIO-3B. Pour ceux exprimant un ARN messager dérivé du gène codant pour la protéine de 42 Kd, le fragment EcoRI de 1330 paires de bases du plasmide pBIO-3-8B a été utilisé comme sonde. Le marquage radioactif de ces 2 sondes grâce à de l'ATP (P32) s'est fait par la technique dite de l'"Oligonucleotide Primed Synthesis" (Ausubel et al. 1987).

Les résultats obtenus ont permis aux inventeurs de vérifier la présence de tous les ARN messagers attendus, ce qui montrait la fonctionnalité des gènes chimériques construits. Ces messagers ont une taille correspondante à celle indiquée dans le tableau 1 majorée de 200 nucléotides environ. Ces nucléotides supplémentaires situés à l'extrémité 3' du mRNA sont dus à la partie transcrite du terminateur Nos jusqu'au signal de polyadénylation et à la queue de poly-A. Il a aussi été observé que le promoteur Nos a une efficacité de transcription de 30 à 50 fois plus faible que le promoteur 35S dans des cellules de betteraves. Ces résultats sont comparables à ceux obtenus sur le tabac et la tomate par Sanders et al (1987).

Bien que la transcription de tous les gènes ait été vérifiée, il était nécessaire de voir si la traduction et la production de la protéine de capside et de la protéine chimérique de 29 Kd a été obtenu dans le cas du vecteur binaire pGA-β-3-1B. Pour cela il a été réalisé un western blot (Ausubel et al, 1987)) sur des protéines solubles extraites à partir de différentes suspensions cellulaires transformées (tampon d'extraction : urée, 9M ; β-mercaptoéthanol, 7,5 %, SDS, 4.5 % ; pH : 6,8). Les résultats sont présentés dans la figure 7 ; la partie supérieure du filtre a été révélée avec des anticorps polyclonaux anti *β*-glucuronidase et la partie inférieure par des anticorps polyclonaux anti-BNYVV. La *β*-glucuronidase est présente dans la majorité des suspensions transformées (bande immunoréagissante 88 Kd). Dix suspensions sur les 11 testées présentent une bande immunoréactive commigrant avec la protéine de capside du BNYVV ainsi qu'une bande d'un poids moléculaire d'environ 29 Kd. L'expression de ces deux protéines est de l'ordre de 0,005 à 0;01 % des protéines solubles totales ; cette quantité atteste de la bonne efficacité de traduction du messager produit. Il est à noter que la quantité de 29 Kd est particulièrement élevée dans la majorité des transformants et peut être supérieure à celui de la protéine de capside. Le taux de readthrough dans les suspensions cellulaires habituées semble être supérieur à celui observé dans des racines de betteraves pour le RNA2 (Ziegler et al, 1985) et sur des betteraves transgéniques (exemple 15).

### EXEMPLE 13 : TEST DE RESISTANCE IN VITRO

### Isolement et purification de protoplastes de betterave (comparatif)

Cinq jours après le repiquage, 2 ml de suspension cellulaire tassée sont digérés dans 20 ml d'enzymes caylase. La composition du cocktail enzymatique est la suivante : 0.25 %. 345 S ; 0.25 % T ; 0.08 % M₂L dissout dans du mannitol 0.7 M contenant 0.08 mM NaH2PO4, 0.03 mM MES et 0.68 mM CaCL2. La pression osmotique est ajustée à 760 mOsmole/Kg et à pH 5,8. après 18 à 18H00 de douce agitation (30 oscillations par minute) à l'obscurité et à 24°C, la suspension est filtrée sur tamis de 100 et 50 µm respectivement. Le filtrat est additionné d'un volume égal d'une solution iso-osmotique de KCI à 470 mM et les protoplastes sont sédimentés à 50 g pendant 5 minutes. Le culot est repris dans du saccharose isoosmotique à 570 mM et centrifugé à 50 g pendant 15 minutes. L'anneau de protoplastes est prélevé, soumis à une deuxième centrifugation dans du saccharose, qui, sédimenté, est lavé deux fois dans du mannitol à 760 mOsmoles/Kg. La viabilité des protoplastes est quantifiée par coloration au diacétate de fluorescéine (Widholm, 1972). On s'assure que la digestion est complète par l'absence de coloration de paroi du calcofluor.

L'utilisation de gradients iso-osmotiques mais de densités différentielles a permis d'obtenir des préparations de protoplastes à très hauts rendements 5 x 10⁶ protoplastes par ml de cellules tassées et complètement débarrasés de débris cellulaires. En moyenne, 90 % des protoplastes sont viables.

### Culture des protoplastes isolés de cellules non transformées et transformées

Le milieu de culture des protoplastes est un milieu de macro et micro éléments de Murashige et Skoog (1962) où le NH₄NO₃ est diminué de moitié et additionné de :
1g/l hydrolysat de caséine
30 g/l saccharose
7,7 mg/l glycine
1,3 mg/l d'acide nicotinique
0,25 ng/l pyridoxine
0,25 n/l Thiamin-HCl
400 mg/l glutamine
25 mg/l glucosamine
1 mg/l 6 benzyl amino purine
1 mg/l acide indole acétique.

La pression osmotique est ajustée à 760 mOsmole/kg avec du mannitol et le pH à 5,8. Le milieu de culture est stérilisé par filtration.

Des protoplastes ont été isolés à partir de suspensions cellulaires transformées et non transformées. Ils ont été mis en culture dans le milieu ci-dessus en présence et en absence de kanamycine à 200 mg/l afin de calculer l'efficacité d'étalement. Les résultats démontrent que les protoplastes isolés à partir de cellules non transformées ne survivent pas sur milieu sélectif contenant 200 mg/l de kanamycine. Ceci est en accord avec l'absence d'échappement dans les expériences de transformation. Aucune différence significative n'a été trouvée entre les efficacités d'étalement de protoplastes issus de cellules transformées en présence et absence de kanamycine. Ceci confirme donc l'absence de cellules non transformées au sein des lignées transformées. Lors d'expériences comparatives qui seront décrites ci-dessous, les protoplastes issus de 4 types de lignées cellulaires (β7 et β14 exprimant la 22 Kd et la 29 Kd, WT, non transformées et β D transformée par le vecteur binaire pGA492) seront mis en culture en absence de pression de sélection.

### Infection de protoplastes de betterave par le BNYVV

L'optimisation de la technique d'infection s'est effectuée sur des protoplastes issus de cellules non transformées. Les inventeurs se sont inspirés de la technique d'infection par le polyéthylène glycol décrit par Samac et al (1983) et de celle par électroporation décrite par Watts et al (1987). Afin d'obtenir des taux élevés d'infectivité, il était impératif d'utiliser des préparations de protoplastes dépourvues de débris, ayant des taux de viabilité supérieurs à 90 % ainsi que des préparations de virus âgées de moins de deux semaines.

La fréquence d'infection a été déterminée par immunofluorescence par une technique indirecte décrite par Maule et al (1980). Les protoplastes infectés ont une fluorescence vert clair caractéristique, dispersée dans le cytoplasme ; les protoplastes non infectés sont marron terne. La figure 8 présente les résultats de 4 expériences d'infection de protoplastes de betterave par la technique au polyéthylène glycol. Il a été constaté qu'après 24 heures de culture environ 60% des protoplastes sont infectés. Ce pourcentage n'évolue pas de façon significative pour des durées de culture supérieures à 24 h. L'infection s'est donc réalisée de façon quasi-synchrone. Cette figure montre aussi que du virus inactivé par congélations et décongélations répétées ne se réplique pas dans les protoplastes de betterave. Dans toutes les expérimentations effectuées, en moyenne 50 % des protoplastes sont infectés.

Les inventeurs ont réussi à introduire des particules virales du BNYVV en utilisant des impulsions électriques (technique d'électroporation). Les inventeurs se sont inspirés de la technique décrite par Watts et al (1987) et ont utilisé un électroporateur à décharge de capacités (Guerche et al, 1987).

L'optimisation de la technique s'est faite en faisant varier la durée d'impulsion délivrée par des condensateurs de différentes capacités et la résistivité du milieu d'électroporation. C'est ainsi que les inventeurs ont vu que deux impulsions de 100 ms délivrées à 15 secs d'intervalle par des condensateurs de capacité 63 µF chargés à 220 V à 1 x 10⁶ protoplastes remis dans du mannitol contenant du CaCl2 à 5 mM en présence de 5 µg de BNYVV permettaient d'avoir un taux de 55 % de protoplastes infectés. L'omission du CaCl2 résulte en une diminution de l'infectivité d'un facteur 3, bien que la viabilité des protoplastes soit augmentée d'un facteur 2. Ceci est illustré sur la figure 9.

### Infection de protoplastes de betteraves issus de cellules transformées et non transformées

Afin de déterminer si la protéine capsidaire (22 kD) du BNYVV et la protéine dérivée (29kD) produite dans les cellules de betterave pouvait inhiber la multiplication du virus, les inventeurs ont inoculé des protoplastes issus de cellules exprimant ou n'exprimant pas ces protéines par le virus. Les expériences ont été réalisées par les techniques au PEG et par l'électroporation. L'infection a été visualisée par immunofluorescence des cellules 30 heures après culture. Il n'a pas été décelé de bruit de fond en immunofluorescence dans les cellules transformées et exprimant la protéine capsidaire avant inoculation.

**TABLEAU 2**

| infection par le BNYVV de protoplastes issus de cellules transformées et non transformées. | | | | | |
|---|---|---|---|---|---|
| | Source protoplastes | | | | |
| | Non transformé | | Transformé | | Protection (%) |
| | | | CP⁺ | CP⁻ | |
| Expérience | P11 | *β*7 | *β*14 | βD | |
| 1 | 30 | | 1 | | 97 |
| 2 | 68 | | 10 | | 85 |
| 3 | 70 | | 4 | | 94 |
| 4 | 27 | | 1 | | 96 |
| 5 | 35 | 5 | | | 86 |
| 6 | 25 | 2 | | | 92 |
| 7 | 37 | 10 | | 36 | 73 |
| 8 | | 12 | | 48 | 75 |

1 x 10⁶ protoplastes ont été inoculés avec 5 µg de BNYVV (F13 ou S2) sauf pour l'expérience 4 où l'inoculum consistait de 30 µg de BNYVV les chiffres représentent le pourcentage de protoplastes fluorescents détectés 30 h après l'inoculation. CP+ et CP- sont des lignées cellulaires transformées exprimant et n'exprimant pas la protéine capsidaire du BNYVV.
Le tableau 2 résume les résultats obtenus avec deux lignées cellulaires (β7 et β14) exprimant fortement deux protéines, et la lignée cellulaire transformée (βD) et la lignée cellulaire non transformée WT. Dans tous les cas, le pourcentage de protoplastes infectés isolés de cellules transformées β7 et β14 est significativement plus faible que celui des protoplastes de cellules non transformées. Les expériences 7 et 8 prouvent bien que la protection est bien due à la présence des protéines 22 Kd et 29 Kd, car le taux d'infection de protoplastes issus des cellules de la lignée βD est comparable à celle des cellules non transformées. Donc le processus de transformation en lui-même ne confère pas de protection à l'infection par le virus. L'expression de protéines étrangères, autres que celles du BNYVV ne confère pas non plus la protection à l'infection par ce même virus. La protection n'est pas abolie lorsque l'on augmente la concentration du virus présente dans l'inoculum. Les profils densitométriques (figure 10) des RNA totaux (isolés de protoplastes β14 et WT infectés) et hybridés avec une sonde cDNA total contre les 4 RNA de BNYVV montrent que ceux-là ne se répliquent pas dans les protoplastes exprimant la protéine capsidaire du virus. La protection a aussi été observée lorsque l'inoculation a été effectuée par électroporation indiquant que la protection est indépendante de la méthode utilisée pour l'inoculation.

Ces résultats démontrent pour la première fois que des protoplastes de betterave exprimant la protéine capsidaire virale du BNYVV et la protéine chimérique dérivée sont protégés contre l'infection par ce même virus.

### EXEMPLE 14 : REGENERATION DE PLANTES A PARTIR DE CALS TRANFORMES

Après un premier passage d'un mois sur M.SB1 + C ou MSB1 + CK, les cals tranformés sont repiqués tous les mois sur MSB1. Après des délais plus ou moins longs, variant d'une semaine à plusieurs mois, des bourgeons et/ou embryons régénèrent sur certains de ces cals.

Il a été observé qu'après transformation les cals ont le même phénotype que la suspension de départ. C'est-à-dire que le type noduleux ou habitué se retrouve après la transformation. En outre, il semble que ce soit les cals tranformés de type noduleux qui aient la plus grande aptitude à la régénération. Ils permettent la régénération de plusieurs structures se développant assez bien et vite en plante, alors que les structures obtenues sur les cals habitués transformés sont très rares, longues et très difficiles à se développer en plante.

Les structures régénérées sont très hétérogènes morphologiquement. Elles sont repiquées, après avoir été coupées à la base au scalpel, sur le milieu MSB0.1 en boîtes de Pétri.

Quand les bourgeons commencent à développer plusieurs feuilles, ils sont remis en multiplication végétative en pots ou boîtes. Les bourgeons les plus développés sont alors mis en enracinement sur MS + ANA 1 mg/l(ANA : acide naphtalène-acétic). Les racines apparaissent de 2 à 6 semaines après.

Dès que les racines sont suffisamment développées, les plantes sont acclimatées en serre dans du terreau universel. Au bout de 3 mois, les plantes sont bien développées (voir photo) et ont perdu la plupart des variations phénotypiques dues à la culture in vitro.

### EXEMPLE 15 : EXPRESSION DE LA PROTEINE DE CAPSIDE ET DE LA PROTEINE CHIMERIQUE DERIVEE DANS LES BETTERAVES TRANSGENIQUES

Bien que sachant les betteraves transgéniques, grâce aux tests de détection du gène codant pour la *β-*glucuronidase, il n'était pas certain que le gène intéressant, à savoir celui codant pour les protéines de capside du BNYVV, était bien exprimé dans ces plantes. Pour le savoir, il faut détecter ces protéines dans les tissus de betterave transformées. Pour détecter les protéines dans les transformants, il a été fait appel à la technique de Western blot (Ausubel et al, 1987). Celle-ci consiste à faire migrer les protéines solubles extraites de broyat de tissus des transformants, sur gel de polyacrylamide en conditions dénaturantes (Laemmli, 1970). Les protéines ainsi séparées sont tranférées par électrotransfert sur une membrane de nitrocellulose. Cette membrane est ensuite hybridée avec des anticorps polyclonaux de lapins dirigés contre le BNYVV. La révélation se fait par addition d'anticorps anti-lapin conjugés à la phosphatase alcaline, qui utilise des substrats chromogènes. La figure 11 montre le type de résultats obtenus. Les extraits de betterave transgéniques contenant le gène codant pour les protéines de capside montrent une bande immunoréactive migrant au même niveau que la protéine de capside du virus. Cette bande n'est pas présente dans les extraits de plante non transformées.De plus, dans les extraits de plantes transformées, on détecte une bande à 29 Kd correspondant à la protéine chimérique dérivée de la 22 Kd par addition de 64 acides aminés, due au "readthrough" (exemple 5). Ces deux protéines de 22 et 29 Kd n'ont pu être détectées que dans des extraits de racines de transformants et pas dans les feuilles. La figure 11 illustre donc l'expression spécifique, dans les racines de betteraves transgéniques, des protéines de 22kD et de 29kD, malgré l'utilisation d'un promoteur constitutif. La fonctionnalité du promoteur dans les parties aériennes de la plante est confirmée par l'expression du gène marqueur GUS dans tous les tissus.

Le BNYVV se transmet et se développe au niveau des racines, il est donc avantageux que les protéines susceptibles d'inhiber le virus soient présentes dans ces organes.

### EXEMPLE 16 : OBTENTION DE GRAINES DE BETTERAVES TRANSGENIQUES

Après 2 à 3 mois d'acclimatation en serre des plantes transgéniques, il a été constaté qu'elles étaient phénotypiquement conformes à la plante mère. A ce stade les plantes possèdent de 10 à 15 feuilles très bien développées, et sont toujours à l'état de rosette. Pour savoir si les plantes obtenues sont tout à fait normales et notamment fertiles, et si le gène introduit est transmis à la descendance, les plantes ont été vernalisées pour induire la montée à graine. Les transformants primaires sont donc placés entre 2 et 7°C à l'obscurité pendant 3 mois, puis en champs en période de jour long. Un mois à un mois et demi après la mise en champ, la hampe florale commence à monter.

La floraison a lieu trois mois environ après la sortie de vernalisation, et les fruits sont mûrs deux mois après.

Quand les glomérules sont bien secs, ils sont récoltés et nettoyés. Les graines sont ainsi prêtes à être semées.

### EXEMPLE 17 : APPLICATION DE LA METHODE DE L'INVENTION A DIFFERENTES VARIETES DE BETA VULGARIS :

Les méthodes de transformation et de régénération décrites dans les exemples 9 à 14 ont été appliquées à des suspensions cellulaires et à des dispersions de cals blancs friables issues de plantes provenant d'une variété "élite" (variété parente d'hybrides commerciaux).

Des plantes transgéniques exprimant la protéine de capside du BNYVV ont été obtenues. Cette expression était spécifique dans les racines. La variété "élite" ayant un environnement génétique différent des autres variétés transformées, l'expression spécifique semble être alors conservée dans l'espèce Beta vulgaris.

La reproductibilité de ces techniques a été vérifiée en appliquant les méthodes de transformation et de régénération de l'invention à d'autres variétés et en d'autres sites géographiques par une autre équipe d'expérimentateurs. Dans chaque cas, des plantes transgéniques ont été obtenues.

### EXEMPLE 18 : AUTOFECONDATIONS ET CROISEMENTS DES PLANTES TRANSGENIQUES RESISTANTES A LA RHIZOMANIE

Des graines transgéniques ont été obtenues à la fois sur des autofécondations des plantes transformées et sur des croisements de ces mêmes plantes avec trois autres lignées de betteraves mâles stériles (une lignée mâle stérile génique monogerme, une lignée mâle stérile génique multigerme et une lignée mâle stérile cytoplasmique).

L'expression spécifique de la protéine de capside et de celle de la protéine de 29kD dans les racines de toutes les plantes transgéniques issues d'autofécondations et de croisements a été obtenue.

Ces résultats attestent que cette expression spécifique est maintenue dans un environnement génétique différent de celui des transformants primaires.

### BIBLIOGRAPHIE

ABEL P.P., NELSON R.S., DE B., HOFFMANN N., ROGERS S.G., FRALEY R.T., BEACHY R.N. (1986) - Science 232 : 738-743.
AKIYOSHI D.E., MORRIS R.O., HINZ R., MISCHKE B.S.; KOSUGE T., GARFINKEL D.J., GORDON M.P., NESTER E.W. (1983) - PNAS 80 : 407-411.
AN G. (1985) - Plant Physiol. 79 : 568-570.
AN G., WATSON B.D., STACHEL S., GORDON M.P., NESTER E.W. (1985) - EMBO J. 4 : 277-284.
AN G. (1986) - Plant Physiol. 81 : 86-91.
AUSUBEL F.M., BRENT R., KINGTON R.E., MOORE D.D., SMITH J.A., SEIDMAN J.G. and STRUHL K. (1987) - Current Protocol in Molecular Biology - Published by Green Publishing Associates and Wiley Interscience.
BEVAN M., FLAVELL R.B., CHILTON M.D. (1983) - Nature 304 : 184-187.
BEDBROOK J.R., CHALEFF R.S., FALCO S.C., MAZUR B.J., YADAV N.S., (1988) - EP-A-0257993
BEVAN M. (1984) - Nucl. ac. Res. 12 : 8711-8721.
BOLIVAR F.; RODRIGUEZ R.L., GREEN P.I., BETLACH M.C., HEYNECKER H.L., BOYER H.W., CROSA J.H., FALKOW S. (1977) - Gene 2 : 95-113.
BOLIVAR F., BACKMAN K. (1979) - Methods in Enzymol. 68 : 245-267.
BOUZOUBAA S., ZIEGLER V., BECK D., GUILLEY H., RICHARDS K., JONARD G. (1986) - J. Gen. Virol. 67 : 1689-1700.
BROADBENT L. (1976) - Ann. Rev. Phytopathol. 14 : 75.
CUOZZO M., O'CONNEL K.M., KANIEWSKI W., FANG R.X., CHUA N.H. and TUMER N.E. (1988) - Biotechnology 6 : 549-557.
DALE P.J., MARKS M.S., BROWN M.M., WOOLSTON C.J., GUNN H.V., MULLINEAUX P.M., LEWIS D.M., KEMP J.M., CHEN D.F., GILMOUR D.M. and FLAVELL R.B. (1989) - Plant Science 63 : 237-245.
DE BLOCK M., BOTTERMAN J., VANDEWIELE M., DOCKX J., THOEN C., GOSSELE V., RAO MOWA N., THOMSON C., VAN MONTAGU M., LEEMANS J., (1987) EMBO J. 6 : n ° 9, 2513-18.
DETREZ C. TETU T., SANGWAN R.S., SANGWAN-NORREEL B.S. (1988) - J. Exp. of Bot. 39 (204) : 917-926.
DITTA G., STANFIELD S., CORBIN D., HELINSKI D.R. (1980) - PNASS 77 : 7347-7351.
FERNOW K.H. (1967) - Phytopathol. 57 : 13-47.
FISCHOFF D.A., et al (1987), BIO/TECHNOLOGY 5 : 807-813.
FRANCK A., GUILLEY H., JONARD G., RICHARDS K., HIRTH L. (1980) - Cell 21 : 285-294.
FREYTAG A.H., ANAND S.C., RAO-ARELLI A.P. and OWENS L.D. (1988) - Plant Cell Reports 7 : 30-34.
GERLACH W.L., LLEWELLYN D. and HASELOFF J. (1987) - Nature 328 : 802-805.
GIELEN J.J.L., et al, Abstracts of 7th International Congress of Plant Tissue and Cell Culture. I.A.P.T.C. Juin 1990, Amsterdam.
GUERCHE P., BELLINI C., LE MOULLEC J.M., CABOCHE M. (1987) - biochimie 69 : 621-628.
HARRISSON D.B., MAYO M.A. and BAULCOMBE (1987) - Nature 328 : 799-802.
HARPSTER M.H., TOWNSEND J.A., JONES J.D.J., BEDBROOK J., DUNSMUIR P.(1988) - Mol. Gen. Genet. 212 : 182-190.
HOEKEMA A., HIRSCH P.R., HOYKAAS P.J.J. and SCHILLPEROOT R.A. (1983) - Nature 303 : 179-180.
HOOD E.E., HELMER G.L., FRALEY R.T., CHILTON M.D. (1986) - J. Bact. 168 : 1291-1301.
JEFFERSON R.A., KAVANAGH T.A. and BEVAN M.W. (1987) - EMBO J. 6 : 3901-3907.
KRENS F.A., ZIJLSTRA C., VAN DEN MOLEN W., JAMAR D. and HUIZING H.J. (1988) - Euphytica S. 185 : 194.
LAEMMLI V. (1970) - Nature 277 : 680-685.
LEWELLEN R.T., SKOYEN I.O. and ERICHSEN A.W. (1987) - in 50th Winter Congress I.I.R.B. Bruxelles 11-12 February 1987, 136-156.
LINDSEY K. and JONES M.G.K. (1989) - Plant Cell Reports 8(2) : 71-74.
LOESH-FRIES L.S., MERLO D., ZIMMENT T., BURHOP L., HILL K., KROHN K., JARNIS N., NELSON S., HALK E. (1987) - EMBO J. 6 : 1845-1851.
MANIATIS T., FRITSCH E.F., SAMBROOK J. (1982) - Molecular cloning. Cold Spring Harbor Laboratory.
MARIANI C., et al (1990), NATURE, vol. 347 : 737-741.
MAULE A.J., BOULTON M.I., WOOD K.R. (1980) - Phytopath Z. 97 : 118-126.
MAXAM A.M. and GILBERT W. (1980) - Methods Enzymol., 65 : 499-560.
MESSING J. (1983) - in methods in Enzymology 101 : 20-78 Academic Press, New York.
MURASHIGE T., SKOOG F. (1962) - Plant Physiol 15 : 473-497.
PERLAK J., et al, (1990) BIO/TECHNOLOGY, vol. 8, 939-943.
PUTZ C. (1977) - J. Gen. Virol. 35 : 397-401.
PUTZ C., RICHARD-MOLARD M. (1984) - C.R. Acad. Agr. de France 70 : 370-378.
RICHARDS K., JONARD G., GUILLEY H., ZIEGLER V. and PUTZ C. (1985) - J. Gen. Virol. 66 : 345-350.
RITCHIE G.A., SHORT K.C., DAVEY M.R. (1989) - J. Exp. Bot. 40(211) : 277-283.
ROTHSTEIN S.J., JORGENSEN R.A., YIN J.C.P., YONG-DI Z., JOHNSON R.C., REZNIKOFF W.S. (1980) - Cold Spring Harbor Symp. Quant. Biol 45 : 99-106.
SALLE G., LE GOZ S. and TOQUET C. (1986) - Physiol. Veg. 24 : 73-83.
SAMAC D.A., NELSON S.E., LOESCH-FRIES L.S. (1983) - Virology 131 : 455-462.
SANDERS P.R., WINTER J.A., BARNASSON A.R., ROGERS S.G., FRALEY R.T. (1987) - Nucl. Acid Res. 15 : 1543-1548.
SANGER F., NICKLEN S., COULSON A.R. (1977) - PNAS 74 : 5463-5467.
SAUNDERS J.W. and DOLEY W.P. (1986) - J. Plant. Physiol. 124 : 473-479.
SCOTT R.J. and DRAPER J. (1987) - Plant Mol. Biol. 8 : 264-274.
TAMADA T. and BABA T. (1973) - Ann. Phytopath. Soc. Japan 39 : 325-332.
TETU T., SANGWAN R.S. and SANGWANN-NORREEL B.S. (1988) - J. Exp. Bot, 38 (188) : 506-517.
TUMER N.E., O'CONNEL K.H., NELSON R.S., BEACHY R.N., FRALEY R.T., SHAH D.H. (1987) - EMBO J. 6 : 1181-1188. TWIGG A.J. and SHERATT D. (1980) - Nature 283 : 216-218.
VAECK M, et al (1987), NATURE, 328, 33-37.
VAN DER WERF S., BREGEGERE F., KOPECKA H., KITAMURA N., ROTHBERG P.G., KOURILOSKY P., WIMMER E., GIRARD M. (1981) - PNAS 78 / 5983-5987.
VAN DUM M.P., BOL J., VAN VLOTEN-DOTING L. (1987) - Virology 159 : 299-305.
VIEIRA J., MESSING J. (1982) - Gene 19 : 259-268.
WATTS J.W., KING J.M., STACEY N.J. (1987) - Virology 157 : 40-46.
WIDHOLM J.M. (1972) - Stain Tech. 47 : 189-194.
YACOUB A. and TEPPER D. (1987) - in 50th Winter Congress I.I.R.B. Bruxelles 11-12 February 1987, 107-308.
ZIEGLER V., RICHARDS K., GUILLEY H., JONARD G., PUTZ C. (1985) - J. Gen. Virol. 66 : 2079-2087.

## Revendications

1. Procédé de transformation de cellules végétales appartenant à l'espèce Beta vulgaris **caractérisé en ce qu'**il comprend la mise en contact d'une dispersion de cals blancs friables dans un milieu de culture cellulaire végétale liquide contenant 0 à environ 3.0 mgL⁻¹ d'une cytokine, avec Agrobacterium tumefaciens contenant un vecteur portant un gène destiné à être introduit dans les cellules végétales, suivie de coculture des cellules végétales et des bactéries pour donner lieu à des cals friables transformés.

2. Procédé selon la revendication 1, **caractérisé en ce qu'**il comprend les étapes successives suivantes
I) induction de cals blancs friables à partir d'explant ;
II) dispersion des cals dans un milieu de culture cellulaire végétale liquide contenant 0 à environ 3.0 mgl-¹ d'une cytokinine ;
III) mise en contact de la dispersion avec Agrobacterium tumefaciens contenant un vecteur portant un gène destiné à être introduit dans les cellules végétales, suivie de coculture des cellules végétales et des bactéries ;
IV) lavage des cellules végétales pour éliminer les bactéries et sélection des cellules transformées sur un milieu sélectif ;
V) culture des cellules transformées sélectionnées pour obtenir des cals friables transformés.

3. Procédé selon l'une quelconque des revendications 1 ou 2, **caractérisé en ce que** les cals blancs friables sont induits à partir de jeunes feuilles, ayant par exemple une longueur de 3 à 5 cm, prelevées d'une plante âgée de moins de trois mois.

4. Procédé selon l'une quelconque des revendications 1 à 3 **caractérisé en ce que** la dispersion des cals s'effectue dans un milieu de culture cellulaire végétale contenant de la 6-benzylaminopurine (BAP), plus particulièrement environ 1mgl⁻¹ BAP.

5. Procédé selon l'une quelconque des revendications précédentes, **caractérisé en ce que** le milieu de culture cellulaire végétale est le milieu de Murashige et Skoog (1962), dit milieu M.S.

6. Procédé selon l'une quelconque des revendications précédentes, **caractérisé en ce que** la coculture de cellules végétales et des bactéries s'effectue pendant 3 jours dans l'obscurité sur un milieu de culture cellulaire végétale tel que le milieu MS, éventuellement additionné de cytokinine, par exemple environ 1 mgl⁻¹ BAP.

7. Procédé selon l'une quelconque des revendications précédentes, **caractérisé en ce que** l'élimination des bactéries s'effectue par lavage des cellules végétales avec un milieu de culture cellulaire végétale contenant un agent bactériostatique inhibant la croissance d'Agrobacterium, par exemple le céfotaxime.

8. Procédé selon l'une quelconque des revendications précédentes, **caractérisé en ce que** la sélection des cellules transformées s'effectue sur un milieu contenant de la kanamycine.

9. Procédé selon l'une quelconque des revendications précédentes, **caractérisé en ce que** la culture des cellules transformées sélectionnées s'effectue sur un milieu de culture solide, tel que le milieu M.S solide, additionné de cytokinine, d'agent bactériostatique et d'agent sélectif.

10. Procédé selon l'une quelconque des revendications 1 à 9 **caractérisée en ce que** le gène destiné à être introduit dans les cellules végétales est choisi parmi un gène conférant un caractère d'intérêt agronomique ou industriel, par exemple un gène de résistance à l'infection par un virus, tel qu'un gène codant pour la protéine de capside du virus BWYV ou du virus BNYVV, un gène conférant une résistance à un herbicide ou à un insecticide, ou encore un gène dont l'expression confère la stérilité mâle

11. Procédé de régénération de bourgeons et/ou d'embryons transgéniques appartenant à l'espèce Beta vulgaris à partir d'explants, **caractérisé en ce qu'**il comprend les étapes successives suivantes :
I) obtention de cals friables transformés selon l'une quelconque des revendications 1 à 10;
II) repiquage des cals transformés sur un milieu de culture contenant 0 à environ 3 mgl⁻¹ d'une cytokinine, et éventuellement un agent bactériostatique et un agent sélectif jusqu'à l'apparition de bourgeons et/ou d'embryons transgéniques.

12. Procédé selon la revendication 11, **caractérisé en ce que** le milieu de culture est le milieu M.S additionné d'environ 1mgl⁻¹ BAP et éventuellement environ 300 mgl⁻¹ cétotaxime et 200 mgl⁻¹ kanamycine.

13. Procédé de régénération de plantes transgéniques appartenant à l'espèce Beta vulgaris, **caractérisé en ce qu'**il comprend les étapes successives suivantes :
I) régénération de bourgeons et/ou d'embryons transgéniques selon le procédé de la revendication 11 ;
II) repiquage des bourgeons et/ou des embryons transgéniques sur un milieu de culture tel que le milieu M.S additionné d'environ 0.1 mgl⁻¹ cytokinine, par exemple de la BAP ;
III) remise des structures régénérées en multiplication végétative suivi d'enracinement sur un milieu de culture tel que le milieu M.S contenant de l'acide naphthalène acétique, par exemple environ 1 mgl⁻¹.

14. Procédé de production de graines de plantes transgéniques appartenant à l'espèce Beta vulgaris **caractérisé en ce que** qu'il comprend les étapes suivantes :
I) régénération de plantes transgéniques selon le procédé de la revendication 13 ;
II) vernalisation des plantes transgéniques et récolte des graines après floraison.

15. Cals friables transformés appartenant à l'espèce Beta vulgaris et pouvant être produits selon l'une quelconque des revendications 1 à 10.

16. Bourgeons et/ou embryons transgéniques appartenant à l'espèce Beta vulgaris et pouvant être produits selon l'une des revendications 11 et 12.

17. Plantes transgéniques appartenant à l'espèce Beta vulgaris et pouvant être produits selon le procédé de la revendication 13.

18. Graines transgéniques de plantes transgéniques appartenant à l'espèce Beta vulgaris et pouvant être produits selon la revendication 14.

19. Plante transgénique appartenant à l'espèce Beta vulgaris et résistante à l'infection par le virus des nervures jaunes et nécrotiques de la betterave à sucre (BNYVV), ladite plante étant transformée d'une manière stable par un fragment d'acide nucléique, dont le produit d'expression est capable de conférer ladite résistance, ledit fragment étant dérivé de l'extrémité 5' de l'ARN2 génomique ou subgénomique du BNYVV, ou du cADN correspondant, ce fragment codant pour au moins une partie des protéines codées par les nucléotides 145 à 3285 de la séquence sauvage de l'ARN2, et étant sous le contrôle d'un promoteur permettant l'expression du fragment dans les cellules de la plante et étant dans l'orientation sens ou antisens.

20. Plante transgénique, selon la revendication 19, dans laquelle ledit fragment code pour au moins une partie de la protéine codée par les nucléotides 145 à 2218 de l'ARN2 ou pour une variante de cette protéine présentant une homologie d'au moins 80 % et comportant l'insertion, la substitution ou la délétion d'acide(s) aminé(s).

21. Plante transgénique, selon la revendication 20, dans laquelle ledit fragment code pour la protéine codée par les nucléotides 145 à 708 et, en outre, pour une partie de la protéine codée par les nucléotides 709 à 2218 de l'ARN2 du BNYVV.

22. Plante transgénique, selon la revendication 21, dans laquelle ledit fragment code pour la protéine codée par les nucléotides 145 à 871 de l'ARN2 du BNYVV.

23. Plante transgénique, selon la revendication 22, dans laquelle ledit fragment est composé par les nucléotides 91 à 871 de l'ARN2 du BNYVV.

24. Plante transgénique, selon la revendication 20, dans laquelle ledit fragment code pour au moins une partie d'une variante de la protéine codée par les nucléotides 145 à 2218, ladite variante se distinguant de la séquence sauvage par la présence de la séquence Glu Asp Leu Pro qui remplace les acides aminés His ALa codés par les nucléotides 253 à 258 de la séquence sauvage.

25. Plante transgénique selon la revendication 24, dans laquelle ledit fragment code pour une partie de la variante et est composé par les nucléotides 91 à 871 de la séquence sauvage, les nucléotides 253 à 258 de la séquence sauvage étant remplacés par ceux codant pour Glu Asp Leu Pro.

26. Plante transgénique, selon la revendication 24, dans laquelle ledit fragment code pour une partie de la variante, ladite partie correspondant à celle codée par les nucléotides 145 à 255 dans la séquence sauvage, les nucléotides 253 à 255 de la séquence sauvage étant remplacés par ceux codant pour Glu.

27. Plante transgénique, selon la revendication 24, dans laquelle ledit fragment code pour une partie de la variante, ladite partie correspondant à celle codée par les nucléotides 256 à 871 dans la séquence sauvage, les nucléotides 256 à 258 de la séquence sauvage étant remplacés par ceux codant pour Asp Leu Pro.

28. Plante transgénique, selon la revendication 20, dans laquelle ledit fragment code pour au moins une partie d'une variante de la protéine codée par les nucléotides 145 à 2218, ladite variante se distinguant de la séquence sauvage par la présence de la séquence Arg Ser Ser Gly au lieu des acides aminés codés par les nucléotides 637 à 651 de la séquence sauvage, la séquence Arg Ser Ser Gly formant le carboxy-terminal de la protéine.

29. Plante transgénique, selon la revendication 28, dans laquelle ledit fragment code pour une partie de la variante et est composé par les nucléotides 91 à 871 de la séquence sauvage, les nucléotides 637 à 654 de la séquence sauvage étant remplacés par ceux codant pour Arg Ser Ser Gly stop.

30. Plante transgénique, selon la revendication 29, dans laquelle ledit fragment code pour une partie de la variante, ladite partie correspondant à celle codée par les nucléotides 144 à 640 de la séquence sauvage.

31. Plante transgénique, selon la revendication 29, dans laquelle ledit fragment code pour une partie de la variante et est composé par les nucléotides 641 à 871 de la séquence sauvage, les nucléotides 641 et 642 de la séquence sauvage étant remplacés par GA et les nucléotides 643 à 654 étant remplacés par ceux codant pour Ser Ser Gly Stop.

32. Plante transgénique, selon la revendication 19 comprenant un fragment de l'extrémité 5' de l'ARN2 subgénomique du BNYVV, ou du cADN correspondant, ledit fragment codant pour au moins une partie de la protéine codée par les nucléotides 2133 à 3285 de l'ARN2, ou pour une variante de cette protéine présentant au moins 80 % d'homologie et comportant l'insertion, la substitution ou la délétion d'acide(s) aminé(s).

33. Plante transgénique, selon la revendication 32 dans laquelle ledit fragment code pour la protéine codée par les nucléotides 2133 à 2774 de l'ARN2 du BNYVV.

34. Plante transgénique, selon la revendication 33 dans laquelle ledit fragment est composé par les nucléotides 2078 à 2774 de l'ARN2 du BNYVV.

35. Plante transgénique, selon l'une quelconque des revendications 19 à 34, **caractérisée en ce qu'**elle exprime la protéine conférant la résistance, et codée par ledit fragment, uniquement dans les racines.

36. Plante transgénique, selon la revendication 35, **caractérisée en ce que** le promoteur contrôlant l'expression de ladite protéine est un promoteur constitutif, par exemple le pNos ou le p35S.

37. Plante transgénique selon la revendication 35, susceptible d'être obtenue par le procédé de la revendication 13.

38. Graines transgéniques de plantes transgéniques selon l'une quelconque des revendications 19 à 37.

39. Graines de plantes transgéniques selon l'une quelconque des revendications 17 ou 19 à 37, comprenant des graines transgéniques, selon la revendication 38.

40. Procédé de production de plantes transgéniques appartenant à l'espèce Beta vulgaris et résistante à l'infection par le BNYVV, ladite plante exprimant, spécifiquement dans les racines, une protéine capable de conférer ladite résistance, ledit procédé comprenant la transformation, selon l'une quelconque des revendications 1 à 10 par l'intermédiaire d'Agrobacterium tumefaciens, de cellules provenant de Beta vulgaris avec un des fragments d'acide nucléique tels que décrits dans l'une quelconque des revendications 19 à 34, la transcription dudit fragment étant sous le contrôle d'un promoteur constitutif tel que le p35S ou le pNos, suivi de la régénération d'une plante transgénique à partir des cellules transformées.

## Claims

1. Method for transforming plant cells belonging to the species Beta vulgaris, **characterized in that** it comprises the bringing into contact of a dispersion of friable white calluses in a liquid plant cell culture medium containing 0 to about 3.0 mgL⁻¹ of a cytokinine, with Agrobacterium tumefaciens containing a vector carrying a gene intended to be introduced into the plant cells, followed by coculturing the plant cells and the bacteria in order to give rise to transformed friable calluses.

2. Method according to Claim 1, **characterized in that** it comprises the following successive steps:
I) induction of friable white calluses from an explant;
II) dispersion of the calluses in a liquid plant cell culture medium containing 0 to about 3.0 mg/l⁻¹ of a cytokinin;
III) bringing the dispersion into contact with Agrobacterium tumefaciens containing a vector carrying a gene intended to be introduced into plant cells, followed by coculturing the plant cells and the bacteria;
IV) washing the plant cells in order to remove the bacteria and selecting the transformed cells on a selective medium;
V) culturing the selected transformed cells in order to obtain transformed friable calluses.

3. Method according to either of Claims 1 and 2, **characterized in that** the friable white calluses are induced from young leaves, being for example 3 to 5 cm in length, collected from a plant less than three months old.

4. Method according to any one of Claims 1 to 3, **characterized in that** the dispersion of the calluses is performed in a plant cell culture medium containing 6-benzylaminopurine (BAP), more particularly about 1 mgl⁻¹ of BAP.

5. Method according to any one of the preceding claims, **characterized in that** the plant cell culture medium is the Murashige and Skoog medium (1962), called M.S. medium.

6. Method according to any one of the preceding claims, **characterized in that** the coculturing of plant cells and bacteria is performed for 3 days in the dark, in a plant cell culture medium such as a MS medium, optionally supplemented with cytokinin, for example about 1 mgl⁻¹ of BAP.

7. Method according to any one of the preceding claims, **characterized in that** the removal of bacteria is performed by washing the plant cells with a plant cell culture medium containing a bacteriostatic agent which inhibits the growth of Agrobacterium, for example cefotaxime.

8. Method according to any one of the preceding claims, **characterized in that** the selection of the transformed cells is performed in a kanamycin-containing medium.

9. Method according to any one of the preceding claims, **characterized in that** the culture of the selected transformed cells is performed on a solid culture medium, such as the solid M.S. medium supplemented with cytokinin, bacteriostatic agent and selective agent.

10. Method according to any one of Claims 1 to 9, **characterized in that** the gene intended to be introduced into the plant cells is chosen from a gene which confers a character of agronomic or industrial benefit, for example a gene for resistance to infection by a virus, such as a gene encoding the capsid protein of the BWYV virus or BNYVV virus, a gene conferring resistance to a herbicide or to an insecticide, or alternatively, a gene whose expression confers male sterility.

11. Method for regenerating transgenic buds and/or embryos belonging to the Beta vulgaris species from explants, **characterized in that** it comprises the following successive steps:
I) producing friable calluses transformed according to any one of Claims 1 to 10;
II) subculturing the transformed calluses in a culture medium containing 0 to about 3 mgl⁻¹ of a cytokinin, and optionally a bacteriostatic agent and a selective agent until the transgenic buds and/or embryos appear.

12. Method according to Claim 11, **characterized in that** the culture medium is the M.S. medium supplemented with about 1 mgl⁻¹ of BAP and optionally about 300 mgl⁻¹ cetotaxime and 200 mgl⁻¹ kanamycin.

13. Method for regenerating transgenic plants belonging to the Beta vulgaris species, **characterized in that** it comprises the following successive steps:
I) regenerating the transgenic buds and/or embryos according to the method of Claim 11;
II) subculturing the transgenic buds and/or embryos in a culture medium such as the M.S. medium supplemented with about 0.1 mgl⁻¹ of cytokinin, for example BAP;
III) bringing back the regenerated structures into vegetative propagation followed by rooting in a culture medium such as the M.S. medium containing naphthaleneacetic acid, for example about 1 mgl⁻¹.

14. Method for producing transgenic plant seeds belonging to the Beta vulgaris species, **characterized in that** it comprises the following steps:
I) regenerating the transgenic plants according to the method of Claim 13;
II) vernalization of the transgenic plants and harvesting the seeds after flowering.

15. Transformed friable calluses belonging to the Beta vulgaris species and which can be produced according to any one of Claims 1 to 10.

16. Transgenic buds and/or embryos belonging to the Beta vulgaris species and which can be produced according to either of Claims 11 and 12.

17. Transgenic plants belonging to the Beta vulgaris species and which can be produced according to the method of Claim 13.

18. Transgenic seeds of transgenic plants belonging to the Beta vulgaris species and which can be produced according to Claim 14.

19. Transgenic plant belonging to the Beta vulgaris species and resistant to infection by the sugar beet necrotic yellow vein virus (BNYVV), the said plant being transformed in a stable manner by a nucleic acid fragment whose expression product is capable of conferring the said resistance, the said fragment being derived from the 5' end of genomic or subgenomic RNA2 of BNYVV, or from the corresponding cDNA, this fragment encoding at least a portion of the proteins encoded by nucleotides 145 to 3285 of the wild type sequence of RNA2, and being under the control of a promoter allowing the expression of the fragment in the plant cells and being in the sense or antisense orientation.

20. Transgenic plant according to Claim 19, in which the said fragment encodes at least a portion of the protein encoded by nucleotides 145 to 2218 of RNA2, or a variant of this protein exhibiting at least 80% homology and comprising the insertion, substitution or deletion of amino acid(s).

21. Transgenic plant according to Claim 20, in which the said fragment encodes a protein encoded by nucleotides 145 to 708 and, in addition, a portion of the protein encoded by nucleotides 709 to 2218 of the BNYVV RNA2.

22. Transgenic plant according to Claim 21, in which the said fragment encodes the protein encoded by nucleotides 145 to 871 of the BNYVV RNA2.

23. Transgenic plant according to Claim 22, in which the said fragment is composed of nucleotides 91 to 871 of BNYVV RNA2.

24. Transgenic plant according to Claim 20, in which the said fragment encodes at least a portion of a variant of the protein encoded by nucleotides 145 to 2218, the said variant being distinguished from the wild type sequence by the presence of the sequence Glu Asp Leu Pro which replaces the amino acids His ALa encoded by nucleotides 253 to 258 of the wild type sequence.

25. Transgenic plant according to Claim 24, in which the said fragment encodes a portion of the variant and consists of nucleotides 91 to 871 of the wild type sequence, nucleotides 253 to 258 of the wild type sequence being replaced by those encoding Glu Asp Leu Pro.

26. Transgenic plant according to Claim 24, in which the said fragment encodes a portion of the variant, the said portion corresponding to that encoded by nucleotides 145 to 255 in the wild type sequence, nucleotides 253 to 255 of the wild type sequence being replaced by those encoding Glu.

27. Transgenic plant according to Claim 24, in which the said fragment encodes a portion of the variant, the said portion corresponding to that encoded by nucleotides 256 to 871 in the wild type sequence, nucleotides 256 to 258 of the wild type sequence being replaced by those encoding Asp Leu Pro.

28. Transgenic plant according to Claim 20, in which the said fragment encodes at least a portion of a variant of the protein encoded by nucleotides 145 to 2218, the said variant being distinguished from the wild type sequence by the presence of the sequence Arg Ser Ser Gly instead of the amino acids encoded by nucleotides 637 to 651 of the wild type sequence, the sequence Arg Ser Ser Gly forming the carboxyl terminals of the protein.

29. Transgenic plant according to Claim 28, in which the said fragment encodes a portion of the variant and consists of nucleotides 91 to 871 of the wild type sequence, nucleotides 637 to 654 of the wild type sequence being replaced by those encoding Arg Ser Ser Gly stop.

30. Transgenic plant according to Claim 29, in which the said fragment encodes a portion of the variant, the said portion corresponding to that encoded by nucleotides 144 to 640 of the wild type sequence.

31. Transgenic plant according to Claim 29, in which the said fragment encodes a portion of the variant and consists of nucleotides 641 to 871 of the wild type sequence, nucleotides 641 and 642 of the wild type sequence being replaced by GA and nucleotides 643 to 654 being replaced by those encoding Ser Ser Gly Stop.

32. Transgenic plant according to Claim 19, comprising a fragment of the 5' end of the subgenomic BNYVV RNA2, or of the corresponding cDNA, the said fragment encoding at least a portion of the protein encoded by nucleotides 2133 to 3285 of RNA2, or a variant of this protein exhibiting at least 80% homology and comprising an insertion, substitution or deletion of amino acid(s).

33. Transgenic plant according to Claim 32, in which the said fragment encodes the protein encoded by nucleotides 2133 to 2774 of BNYVV RNA2.

34. Transgenic plant according to Claim 33, in which the said fragment is composed of nucleotides 2078 to 2774 of BNYVV RNA2.

35. Transgenic plant according to any one of claims 19 to 34, **characterized in that** it expresses the protein conferring resistance and encoded by the said fragment only in the roots.

36. Transgenic plant according to Claim 35, **characterized in that** the promoter controlling the expression of the said protein is a constitutive promoter, for example pNos or p35S.

37. Transgenic plant according to claim 35, capable of being obtained by the method of Claim 13.

38. Transgenic seeds of transgenic plants according to any one of Claims 19 to 37.

39. Seeds of transgenic plants according to any one of Claims 17 or 19 to 37, comprising transgenic seeds according to Claim 38.

40. Method for producing transgenic plants belonging to the Beta vulgaris species and resistant to infection by BNYVV, the said plant expressing, specifically in the roots, a protein capable of conferring the said resistance, the said method comprising the transformation, according to any one of Claims 1 to 10, via Agrobacterium tumefaciens, of cells derived from Beta vulgaris with one of the nucleic acid fragments as described in any one of Claims 19 to 34, the transcription of the said fragment being under the control of a constitutive promoter such as p35S or pNos, followed by the regeneration of a transgenic plant from the transformed cells.

## Patentansprüche

1. Verfahren zur Transformation von Pflanzenzellen, die der Spezies *Beta vulgaris* angehören, **dadurch gekennzeichnet, daß** dieses umfaßt: das Zusammenbringen einer Dispersion von Weißen brüchigen Kalli in einem flüssigen Pflanzenzellenkulturmedium, enthaltend 0 bis etwa 3,0 mg•l⁻¹ eines Cytokins, mit *Agrobacterium tumefaciens* enthaltend einen Vektor, der ein in die Pflanzenzellen einzubringendes Gen trägt, gefolgt von gemeinsamem Kultivieren der Pflanzenzellen und Bakterien, um transformierte brüchige Kalli zu ergeben.

2. Verfahren nach Anspruch 1, **dadurch gekennzeichnet, daß** es die folgenden sukzessiven Schritte umfaßt:
I) Induktion von weißen brüchigen Kalli aus Explantat;
II) Dispersion der Kalli in einem flüssigen Pflanzenzellenkulturmedium, enthaltend 0 bis etwa 3,0 mg•l⁻¹ eines Cytokinins;
III) Zusammenbringen der Zelldispersion mit *Agrobacterium tumefaciens*, enthaltend einen Vektor, der ein in die Pflanzenzellen einzubringendes Gen trägt, gefolgt von gemeinsamem Kultivieren der Pflanzenzellen und Bakterien;
IV) Waschen der Pflanzenzellen zur Abtrennung der Bakterien und Selektion der transformierten Zellen auf einem selektiven Medium;
V) Kultivieren der transformierten selektionierten Zellen, um transformierte brüchige Kalli zu erhalten.

3. Verfahren nach irgendeinem der Ansprüche 1 oder 2, **dadurch gekennzeichnet, daß** die weißen brüchigen Kalli aus jungen Blättern induziert werden, die beispielsweise eine Länge von 3 bis 5 cm aufweisen und einer wenigstens drei Monate alten Pflanze entnommen wurden.

4. Verfahren nach irgendeinem der Ansprüche 1 bis 3, **dadurch gekennzeichnet, daß** die Dispersion der Kalli in einem Pflanzenzellenkulturmedium durchgeführt wird, das 6-Benzylaminopurin (BAP) enthält, insbesondere etwa 1 mg•l⁻¹ BAP.

5. Verfahren nach irgendeinem der vorstehenden Ansprüche, **dadurch gekennzeichnet, daß** es sich bei dem Pflanzenzellenkulturmedium um das Medium von Murashige und Skoog (1962) handelt, das MS-Medium genannt wird.

6. Verfahren nach irgendeinem der vorstehenden Ansprüche, **dadurch gekennzeichnet, daß** das gemeinsame Kultivieren von Pflanzenzellen und Bakterien über drei Tage im Dunklen auf einem Pflanzenzellenkulturmedium wie etwa dem MS-Medium durchgeführt wird, das gegebenenfalls mit einem Cytokinin versetzt ist, beispielsweise etwa 1 mg•l⁻¹ BAP.

7. Verfahren nach irgendeinem der vorstehenden Ansprüche, **dadurch gekennzeichnet, daß** die Abtrennung der Bakterien durch Waschen der Pflanzenzellen mit einem Pflanzenzellenkulturmedium vorgenommen wird, das ein Bakteriostatikum enthält, welches das Wachstum von *Agrobacterium* hemmt, zum Beispiel Cefotaxim.

8. Verfahren nach irgendeinem der vorstehenden Ansprüche, **dadurch gekennzeichnet, daß** die Selektion der transformierten Zellen auf einem Kanamycin enthaltenden Medium durchgeführt wird.

9. Verfahren nach irgendeinem der vorstehenden Ansprüche, **dadurch gekennzeichnet, daß** die Kultivierung der transformierten selektionierten Zellen auf einem festen Kulturmedium durchgeführt wird, etwa festem MS-Medium, das versetzt ist mit einem Cytokinin, einem Bakteriostatikum und einem selektiven Agens.

10. Verfahren nach irgendeinem der Ansprüche 1 bis 9, **dadurch gekennzeichnet, daß** das in die Pflanzenzellen einzubringende Gen ausgewählt ist aus einem Gen, das landwirtschaftlich oder industriell interessante Eigenschaften verleiht, zum Beispiel ein Gen für die Resistenz gegenüber Virusinfektionen, etwa ein Gen, das für das Kapsidprotein des Virus BWYV oder des Virus BNYVV codiert, ein Gen, das Resistenz gegenüber Herbiziden oder Insektiziden verleiht, oder ein Gen, dessen Expression männliche Sterilität verleiht.

11. Verfahren zur Regeneration von der Spezies *Beta vulgaris* angehörenden transgenen Sprossen und/oder Embryonen aus Explantaten, **dadurch gekennzeichnet, daß** es die folgenden sukzessiven Schritte umfaßt:
I) Gewinnung von transformierten brüchigen Kalli nach irgendeinem der Ansprüche 1 bis 10;
II) Umsetzen der transformierten Kalli auf ein Medium, enthaltend 0 bis etwa 3 mg•l⁻¹ eines Cytokinins und gegebenenfalls ein Bakteriostatikum und ein selektives Agens, bis zum Auftreten von transgenen Sprossen und/oder Embryonen.

12. Verfahren nach Anspruch 11, **dadurch gekennzeichnet, daß** es sich bei dem Kulturmedium um das MS-Medium handelt, versetzt mit etwa 1 mg•l⁻¹ BAP und gegebenenfalls etwa 300 mg•l⁻¹ Cefotaxim und 200 mg•l⁻¹ Kanamycin.

13. Verfahren zur Regeneration von transgenen Pflanzen, die der Spezies *Beta vulgaris* angehören, **dadurch gekennzeichnet, daß** es die folgenden sukzessiven Schritte umfaßt:
I) Regeneration von transgenen Sprossen und/oder Embryonen gemäß dem Verfahren nach Anspruch 11,
II) Umsetzen der transgenen Sprossen und/oder Embryonen auf ein Kulturmedium wie etwa das MS-Medium, das mit etwa 0,1 mg•l⁻¹ eines Cytokinins, beispielsweise BAP, versetzt ist;
III) Rückführung der regenerierten Strukturen in die vegetative Vermehrung, gefolgt von Einwurzelung auf einem Kulturmedium wie etwa dem MS-Medium, das Naphthalinessigsäure enthält, beispielsweise etwa 1 mg•l⁻¹.

14. Verfahren zur Herstellung von Samen transgener Pflanzen, die der Spezies *Beta vulgaris* angehören, **dadurch gekennzeichnet, daß** es die folgenden sukzessiven Schritte umfaßt:
I) Regeneration von transgenen Pflanzen gemäß dem Verfahren nach Anspruch 13;
II) Vernalisation der transgenen Pflanzen und Gewinnung der Samen nach der Blüte.

15. Transformierte brüchige Kalli, die der Spezies *Beta vulgaris* angehören und nach irgendeinem der Ansprüche 1 bis 10 hergestellt werden können.

16. Transgene Sprossen und/oder Embryonen, die der Spezies *Beta vulgaris* angehören und nach einem der Ansprüche 11 und 12 hergestellt werden können.

17. Transgene Pflanzen, die der Spezies *Beta vulgaris* angehören und nach dem Verfahren von Anspruch 13 hergestellt werden können.

18. Transgene Samen von transgenen Pflanzen, die der Spezies *Beta vulgaris* angehören und nach dem Verfahren von Anspruch 14 hergestellt werden können.

19. Transgene Pflanze, die der Spezies *Beta vulgaris* angehört und gegenüber Infektion durch den Virus der gelben nekrotischen Adern der Zuckerrübe (BNYVV) resistent ist, wobei die Pflanze in stabiler Weise durch ein Nucleinsäure-Fragment transformiert ist, dessen Expressionsprodukt imstande ist, diese Resistenz zu verleihen, wobei dieses Fragment abgeleitet ist vom 5'-Ende genomischer oder subgenomischer RNA2 des BNYVV oder von der entsprechenden cDNA, wobei dieses Fragment für wenigstens einen Teil der Proteine codiert, die durch die Nucleotide 145 bis 3285 der RNA2-Wildtypsequenz codiert werden, und unter der Kontrolle eines Promotors steht, der die Expression des Fragments in den Pflanzenzellen ermöglicht, und die Orientierung Sinn oder Antisense aufweist.

20. Transgene Pflanze nach Anspruch 19, wobei das Fragment für wenigstens einen Teil des Proteins codiert, das durch die Nucleotide 145 bis 2218 der RNA2 codiert wird, oder für eine Variante dieses Proteins, das eine Homologie von wenigstens 80% aufweist und Insertion, Substitution oder Deletion von Aminosäure(n) umfaßt.

21. Transgene Pflanze nach Anspruch 20, wobei das Fragment für das Protein codiert, das durch die Nucleotide 145 bis 708 codiert wird, und außerdem für einen Teil des Proteins, das durch die Nucleotide 709 bis 2218 der RNA2 des BNYVV codiert wird.

22. Transgene Pflanze nach Anspruch 21, wobei das Fragment für das Protein codiert, das durch die Nucleotide 145 bis 871 der RNA2 des BNYVV codiert wird.

23. Transgene Pflanze nach Anspruch 22, wobei das Fragment aus den Nucleotiden 91 bis 871 der RNA2 des BNYVV zusammengesetzt ist.

24. Transgene Pflanze nach Anspruch 20, wobei das Fragment für wenigstens einen Teil einer Variante des Proteins codiert, das durch die Nucleotide 145 bis 2218 codiert wird, wobei sich die Variante von der Wildtypsequenz durch das Vorhandensein der Sequenz Glu-Asp-Leu-Pro unterscheidet, die die Aminosäuren His-Ala ersetzt, die durch die Nucleotide 253 bis 258 der Wildtypsequenz codiert werden.

25. Transgene Pflanze nach Anspruch 24, wobei das Fragment für einen Teil der Variante codiert und zusammengesetzt ist aus den Nucleotiden 91 bis 871 der Wildtypsequenz, wobei die Nucleotide 253 bis 258 der Wildtypsequenz durch jene ersetzt sind, die für Glu-Asp-Leu-Pro codieren.

26. Transgene Pflanze nach Anspruch 24, wobei das Fragment für einen Teil der Variante codiert, wobei dieser Teil jenem entspricht, der durch die Nucleotide 145 bis 255 in der Wildtypsequenz codiert wird, und wobei die Nucleotide 253 bis 255 der Wildtypsequenz durch jene ersetzt sind, die für Glu codieren.

27. Transgene Pflanze nach Anspruch 24, wobei das Fragment für einen Teil der Variante codiert, wobei dieser Teil jenem entspricht, der durch die Nucleotide 256 bis 871 in der Wildtypsequenz codiert wird, und wobei die Nucleotide 256 bis 258 der Wildtypsequenz durch jene ersetzt sind, die für Asp-Leu-Pro codieren.

28. Transgene Pflanze nach Anspruch 20, wobei das Fragment für wenigstens einen Teil einer Variante des Proteins codiert, das durch die Nucleotide 145 bis 2218 codiert wird, wobei sich die Variante von der Wildtypsequenz durch das Vorhandensein der Sequenz Arg-Ser-Ser-Gly anstelle der Aminosäuren unterscheidet, die durch die Nucleotide 637 bis 651 der Wildtypsequenz codiert werden, und wobei die Sequenz Arg-Ser-Ser-Gly das Carboxyl-terminale Ende des Proteins bildet.

29. Transgene Pflanze nach Anspruch 28, wobei das Fragment für einen Teil der Variante codiert und zusammengesetzt ist aus den Nucleotiden 91 bis 871 der Wildtypsequenz, wobei die Nucleotide 637 bis 654 der Wildtypsequenz durch jene ersetzt sind, die für Arg-Ser-Ser-Gly-Stop codieren.

30. Transgene Pflanze nach Anspruch 29, wobei das Fragment für einen Teil der Variante codiert, wobei dieser Teil jenem entspricht, der durch die Nucleotide 144 bis 640 der Wildtypsequenz codiert wird.

31. Transgene Pflanze nach Anspruch 29, wobei das Fragment für einen Teil der Variante codiert und zusammengesetzt ist aus den Nucleotiden 641 bis 871 der Wildtypsequenz, wobei die Nucleotide 641 und 642 der Wildtypsequenz durch GA und die Nucleotide 643 bis 654 durch jene ersetzt sind, die für Ser-Ser-Gly-Stop codieren.

32. Transgene Pflanze nach Anspruch 19, enthaltend ein Fragment des 5'-Endes der subgenomischen RNA2 des BNYVV oder der entsprechenden cDNA, wobei das Fragment für wenigstens einen Teil des Proteins codiert, das durch die Nucleotide 2133 bis 3285 der RNA2 codiert wird, oder für eine Variante dieses Proteins, das eine Homologie von wenigstens 80% aufweist und Insertion, Substitution oder Deletion von Aminosäure(n) umfaßt.

33. Transgene Pflanze nach Anspruch 32, wobei das Fragment für das Protein codiert, das durch die Nucleotide 2133 bis 2774 der RNA2 des BNYVV codiert wird.

34. Transgene Pflanze nach Anspruch 33, wobei das Fragment aus den Nucleotiden 2078 bis 2774 der RNA2 des BNYVV zusammengesetzt ist.

35. Transgene Pflanze nach irgendeinem der Ansprüche 19 bis 34, **dadurch gekennzeichnet, daß** diese das Resistenz verleihende und durch das Fragment codierte Protein ausschließlich in den Wurzeln exprimiert.

36. Transgene Pflanze nach Anspruch 35, **dadurch gekennzeichnet, daß** der die Expression des Proteins kontrollierende Promotor ein konstitutiver Promotor ist, beispielsweise pNOS oder p35S.

37. Transgene Pflanze nach Anspruch 35, die sich gemäß dem Verfahren nach Anspruch 13 erhalten läßt.

38. Transgene Samen von transgenen Pflanzen nach irgendeinem der Ansprüche 19 bis 37.

39. Samen von transgenen Pflanzen nach irgendeinem der Ansprüche 17 oder 19 bis 37, die transgene Samen nach Anspruch 38 enthalten.

40. Verfahren zur Herstellung transgener Pflanzen, die der Spezies *Beta vulgaris* angehören und gegen BNYVV-Infektion resistent sind, wobei die Pflanze speziell in den Wurzeln ein Protein exprimiert, das diese Resistenz zu verleihen vermag, wobei das verfahren umfaßt: die Transformation, nach irgendeinem der Ansprüche 1 bis 10, von Zellen, die aus *Beta vulgaris* stammen, mit einem der in irgendeinem der Ansprüche 19 bis 34 beschriebenen Nucleinsäure-Fragmente durch den Vermittler *Agrobacterium tumefaciens*, wobei die Transkription des Fragments der Kontrolle eines konstitutiven Promotors wie etwa p35S oder pNOS unterliegt, gefolgt von der Regeneration einer transgenen Pflanze aus den transformierten Zellen.
